(19) Europäisches Patentamt / European Patent Office / Office européen des brevets

(11) **EP 2 490 288 A1**

(12) **EUROPEAN PATENT APPLICATION**
published in accordance with Art. 153(4) EPC

(43) Date of publication:
22.08.2012 Bulletin 2012/34

(51) Int Cl.:
*H01M 8/04* (2006.01)   *H01M 8/02* (2006.01)
*H01M 8/08* (2006.01)

(21) Application number: 10823274.5

(22) Date of filing: 22.09.2010

(86) International application number:
PCT/JP2010/066403

(87) International publication number:
WO 2011/046006 (21.04.2011 Gazette 2011/16)

(84) Designated Contracting States:
AL AT BE BG CH CY CZ DE DK EE ES FI FR GB
GR HR HU IE IS IT LI LT LU LV MC MK MT NL NO
PL PT RO SE SI SK SM TR

(30) Priority: 16.10.2009 JP 2009239245
23.02.2010 JP 2010037566
12.03.2010 JP 2010055770

(71) Applicant: Olympus Corporation
Shibuya-ku
Tokyo 151-0072 (JP)
(72) Inventors:
• KISHIDA, Takayuki
Tokyo 151-0072 (JP)
• AKAGI, Toshimasa
Tokyo 151-0072 (JP)
• HIBINO, Hiroki
Tokyo 151-0072 (JP)

(74) Representative: Schicker, Silvia
Wuesthoff & Wuesthoff
Patent- und Rechtsanwälte
Schweigerstrasse 2
81541 München (DE)

(54) **FUEL CELL, BATTERY, AND ELECTRODE FOR FUEL CELL**

(57) Provided is a fuel cell for being implanted which enables a long time operation while reducing its size so as to be implanted in a living body. The fuel cell 201 to be adopted includes: a container 211 which contains a fuel such as glucose and an electrolyte solution therein; a pair of electrodes which are arranged in the container 211 and have a noble metal catalyst fixed thereon; an aeration portion 27 which is formed on at least one part of the outer surface of the container 211 and has air permeability and waterproofness; and septa 218 and 219 for injecting the fuel from the outside into the container 211 or discharging it from the container 211.

FIG. 1

EP 2 490 288 A1

## Description

{Technical Field}

The present invention relates to a fuel cell, a battery and an electrode for the fuel cell.

{Background Art}

A biofuel cell is conventionally known which uses sugar and alcohol as fuels and assumes a long time operation in a living body (for instance, see Patent Literature 1). In addition, a small-sized fuel cell assumed to be used for a cardiac pacemaker is known (for instance, see Patent Literature 2).

The fuel cell disclosed in Patent Literature 1 uses sugar and alcohol as fuels when generating an electric power, and uses an enzyme which is a protein that oxidizes the fuels, as an electrode. In addition, the fuel cell has a plurality of fuel cell units provided therein which are isolated from each other by a biodegradable high-polymer, so as to generate an electric power over a long period of time. Partition walls for isolating these fuel cell units are constituted by biodegradable high-polymers having different degradation periods of time from each other, and result in collapsing one by one after the fuel cell has started its electric power generation. Thereby, the plurality of the fuel cell units start the electric power generation one by one, and consequently can be operated for a long period.

The fuel cell disclosed in Patent Literature 2 is structured so as to use an enzyme for an electrode, which is highly compatible with a living body, and uses body fluid or blood in the living body as a fuel, as a fuel cell to be used for the cardiac pacemaker to be implanted in the living body.

In a battery having the plurality of the cells, the electrode arranged in each cell needs to be dipped in each individual electrolyte, in order that these cells are connected in series. This is because electric charges migrate between the plurality of the cells on the condition that the electrode in each cell is dipped in a common electrolyte, and a serial voltage cannot be obtained when the cells are connected in series.

In order to avoid the above described problems, a structure is adopted for the fuel cell as well, in which each cell is surrounded by an independent case so that each cell connected in series can be constituted by each independent electrolyte. It becomes necessary in a fuel cell which uses a liquid as a fuel to individually inject a fuel liquid into each independent cell, in order to supply the fuel liquid into the independent case. In order to save such a labor, a method is conventionally disclosed which injects the fuel liquid into the case and then divides the inner part of the case into a plurality of cells with an air valve (for instance, see Non-Patent Literature 1).

In addition, in the fuel cell, a membrane-electrode assembly (Membrane Electrode Assembly; MEA) is conventionally used, in which a pair of electrodes are arranged so as to sandwich a proton conductor, and are integrally formed so that the electrodes and the proton conductor closely come in contact with each other (for instance, see Patent Literature 3). A material having a porous structure is used for the electrode, in order to increase the area of the electrode to contact air or the fuel.

{Citation List}

{Patent Literature}

{PTT, 1 }
Japanese Unexamined Patent Application, Publication No. 2008-270206
{PTL 2}
the Japanese Domestic Re-publication of PCT International Publication No. WO 2004/012811
{PTL 3}
Japanese Unexamined Patent Application, Publication No. 2008-282586

{Non Patent Literature}

{NPL 1}
Matsuhiko Nishizawa (Tohoku University) "Information energy device having biological function", Tohoku University Global COE booklet, July, 2009

{Summary of Invention}

{Technical Problem}

In the fuel cell disclosed in Patent Literature 1, a long time operation of the fuel cell itself is not achieved, and accordingly the fuel cell avoids the problem by connecting a plurality of fuel cell units to each other and having the fuel cell units provided therein. For this reason, the fuel cell needs to have many fuel cell units provided therein according to the operation period, and is difficult to reduce the size when being commercialized. It is an indispensable requirement for the fuel cell to reduce the size, in order to be used particularly in a form of being implanted in a living body, and accordingly the fuel cell is difficult to be used as an implant. In addition, as the fuel cell has more fuel cell units provided therein so as to be operated for a long time, such new more problems occur that the factors of the failure increase and variations of the performance among each fuel cell unit increase.

A reason why the fuel cell disclosed in Patent Literature 1 cannot achieve the long time operation is because an enzyme is used as an electrode in generating an electric power from sugar and alcohol. Because the enzyme is an organic matter originally exists in the living body, the enzyme has high compatibility with the living body, but on the contrary, has also high degradability in the living body, and is difficult to show the stability for a long time.

In addition, this enzyme lowers the activity remarkably caused by dissolved oxygen or the like in the living body. In the case of the enzyme used in the living body, new enzyme can be always supplied in a similar way to metabolism of the living body, but in the case of the enzyme fixed on the electrode, if the activity has been lowered by the dissolved oxygen or other organic matters, it becomes difficult to generate the electric power at the time point.

On the other hand, the fuel cell disclosed in Patent Literature 2 uses body fluid or blood in the living body as a fuel, but there are actually many substances such as proteins, organic matters, lipids and electrolytes other than the sugar which is used as a fuel, in the body fluid and the blood, and these substances adsorb to the electrode to result in causing the deterioration of the activity of the electrode.

Further, when the blood is used, the substances having adsorbed to the electrode or the electrode itself may cause thrombus by working as a trigger. Accordingly, the blood cannot be easily used. In this point, Patent Literature 2 does not describe a measure against the phenomenon that the unnecessary organic matters such as protein adsorb to the electrode and causes the deterioration of the activity, and actually the fuel cell is difficult to be operated for a long time similarly to that in Patent Literature 1.

Furthermore, a technology disclosed in Non-Patent Literature 1 has such inconveniences that an electric charge migrates between the cells because an air valve for dividing each cell has low water-tightness, and a serial voltage cannot be occasionally obtained in the case of serial connection.

Moreover, a technology disclosed in Patent Literature 3 has such a problem that in a negative electrode for oxidizing the fuel, out of the electrodes, the fuel becomes more difficult to diffuse as the position becomes deeper from the surface of the negative electrode, and accordingly the fuel which has been already oxidized stays there and a new fuel is not smoothly supplied. Particularly when a sugar solution is used as a fuel, this problem remarkably appears because the sugar solution has higher viscosity than that of hydrogen gas and alcohol and is more difficult to diffuse. In other words, there is such a problem that the oxidation reaction of the sugar becomes difficult to occur with the passage of the time, thereby power generation efficiency is lowered and an output current decreases.

A first object of the present invention is to provide a fuel cell for being implanted which enables a long time operation while reducing its size so as to be implanted in the living body.

A second object of the present invention is to provide a battery having a plurality of cells into which a fuel liquid can be easily injected and between which an electric charge can be prevented from migrating.

A third object of the present invention is to provide an electrode for a fuel cell, which can stably supply an output current while maintaining the power generation efficiency even when a sugar solution is used as a fuel, and the fuel cell provided with the same.

{Solution to Problem}

In order to achieve the above described objects, the present invention adopts the following solutions. A first aspect according to the present invention is a fuel cell which includes: a container that contains an electrolyte solution therein; a pair of electrodes arranged in the container; an aeration portion that is formed on at least one part of an outer surface of the container and has air permeability and waterproofness; and an injection/discharge port for injecting a fuel from the outside into the container or discharging the fuel from the container.

According to the first aspect according to the present invention, a fuel such as glucose is injected into the container containing an electrolyte solution, by a syringe or the like through the injection/discharge port, and the electric power is generated in the container with the use of this fuel. Specifically, the fuel such as the glucose that has been injected into the container emits an electron in one electrode, while using a noble metal such as gold, silver and platinum as a catalyst, which has been fixed on the surface of the electrode, and also produces a hydrogen ion (oxidation). The electron which has been emitted in the one electrode is sent to the other electrode through a wire which electrically connects the pair of the electrodes. The hydrogen ion migrates to a vicinity of the other electrode in the electrolyte solution in the container. Thereby, the hydrogen ion which has come to the other electrode while migrating in the electrolyte solution, the electron which has been sent from the one electrode and the oxygen which has been supplied into the container through the aeration portion react with each other on the other electrode to produce water (reduction). As described above, the electric power is generated by the oxidation in the one electrode and the reduction in the other electrode, and the electric power can be supplied to equipment such as a pacemaker which is electrically connected to these electrodes.

In this case, by fixing a single noble metal or a plurality of noble metals on the electrode, these noble metals can be functioned as a catalyst, and the electrolyte solution can be made to neutrality or weak acidity that is equivalent to that of body fluid, which can decrease damage to the living body even in the case in which the electrolyte solution has temporarily leaked from the container that has been implanted in the living body. Particularly, if two or more types of the noble metals are used, the sugar is oxidized by an electric field action that has been generated between the different types of the noble metals, and the electric power is easily generated.

Here, when the electric power is generated by using the sugar in the living body as a fuel, an organic matter such as protein and lipid in the living body adsorbs even to the electrode having the noble metal fixed thereon, and the activity of the electrode results in being immediately lost. In contrast to this, the fuel cell according to the present invention can supply a fuel having high purity into the container from the outside, accordingly can prevent the organic matter from adsorbing to the electrode, can reduce the deterioration of the activity of the catalyst, and can be operated for a long time.

In addition, the electric power can be continuously generated by replenishing the fuel having high purity from the outside, though the fuel is consumed by the generation of the electric power. In other words, by enabling the fuel to be supplied from the outside through the injection/discharge port, the size of the container can be reduced and the whole fuel cell can be down-sized.

In the above described first aspect, the fuel cell may also include a storage portion for storing a fuel supplied from the outside therein, and a flow channel for connecting the container to the storage portion.

When the fuel cell has the storage portion for storing the fuel provided therein, the fuel can be appropriately supplied into the container and the electric power can be generated for a long time. In addition, the frequency at which the fuel is injected from the injection/discharge port can be decreased, and when the fuel cell has been implanted in the living body, the burden to a patient can be mitigated when the fuel is injected or discharged. The container and the storage portion may be formed so as to be an integrated type or may also be formed so as to be a separated type.

In the above described first aspect, the injection/discharge port may also be provided on an outer surface of at least one of the container and the storage portion.

In order to inject the fuel into the container or the storage portion which has been implanted in the living body from the outside or discharge it from the container or the storage portion, a syringe needle needs to be repeatedly inserted into the body of the patient. Accordingly, when the injection/discharge port of the fuel is provided on at least one of the container or the storage portion, the burden to the patient can be mitigated when the fuel is injected or discharged. In addition, when this injection/discharge port is subcutaneously implanted, the port is not exposed to the outside of the body and a sanitary state can be enhanced.

In the above described first aspect, a partition wall may be provided in an inner part of the storage portion so as to divide the storage portion into one face side in which the injection/discharge port is provided and another face side which opposes the one face and so as to be opened in an end edge, and the flow channel may also be connected to each division.

When the inner part of the storage portion is divided by the partition wall into the one face side in which the injection/ discharge port is provided and the other face side which opposes the one face, the temperature of a fuel in the other face side (inner body side) can be kept higher than that of a fuel in the one face side (body surface side) in which the injection/discharge port is provided, when the storage portion is implanted in the living body. When the temperature difference is thus formed between the fuel in the inner body side and the fuel in the body surface side, convection of the fuel in the storage portion is promoted, and the fuel can be supplied to the container from the storage portion through the flow channel by using this convection.

In the above described first aspect, the fuel cell may also have a heat exchanger which exchanges heat between the outside and the inside of the storage portion, provided on an outer surface of the storage portion.

By having the heat exchanger which exchanges the heat between the outside and the inside of the storage portion provided on the outer surface of the storage portion, the fuel cell can efficiently form the temperature difference between the fuel in the inner body side and the fuel in the body surface side, promotes the convection of the fuel in the storage portion and can efficiently supply the fuel to the container from the storage portion.

In the above described first aspect, the aeration portion may also be formed of a carbon fluoride resin.

When the aeration portion is formed of the carbon fluoride resin, for instance, such as ethylene tetrafluoride, the fuel cell can adequately supply oxygen to the electrode in the container while preventing leakage of the electrolyte solution from the container, and can efficiently conduct a reduction reaction on the electrode.

In the above described first aspect, a wall of the container may be formed of a carbon fluoride resin, and the aeration portion may also be a portion in which the wall of the container is formed so as to be locally thin.

By being formed in this way, the fuel cell can increase the amount of oxygen to be supplied to the electrode in the container while preventing the leakage of the electrolyte solution from the container, and can enhance the reduction reaction on the electrode, in other words, the efficiency of the power generation. In addition, the fuel cell can eliminate an interface between the container and the aeration portion, and can enhance safety when the container is implanted in the living body.

A second aspect according to the present invention is a battery which includes: a container that contains an electrolyte fluid therein; a partition wall for dividing the container and forming a plurality of cells in the container; a positive electrode and a negative electrode arranged in each of the cells, respectively; an injection port provided in the container, through which the electrolyte fluid is injected into the container from the outside; a continuous hole which is provided in the partition wall and makes each of the cells communicate with each other; and a flow-channel opening/closing portion which is provided in the continuous hole and opens/closes the flow channel between each of the cells, wherein the flow-channel opening/closing portion opens the flow channels between each of the cells when the electrolyte fluid is injected into the container, and closes the flow channels between each of the cells after the electrolyte fluid has been injected into the container.

According to the second aspect according to the present invention, the electrolyte fluid is injected into the container through the injection port, and thereby an electric power is generated in the container. Specifically, in the negative electrode, a substance such as hydrogen and metal emits an electron, and also elutes in the electrolyte fluid in the container as a positive ion (oxidation). The electron which has been emitted in the negative electrode is sent to the positive electrode through a wire for electrically connecting the negative electrode with the positive electrode. The positive ion migrates to the vicinity of the positive electrode in the electrolyte fluid in the container. Thereby, the positive ion which has migrated in the electrolyte fluid and the electron which has been sent from the negative electrode react with each other on the positive electrode to produce a substance such as hydrogen and metal (reduction). As described above, an electric power is generated by the oxidation to be conducted in the negative electrode and the reduction to be conducted in the positive electrode, and the electric power is supplied to electronic equipment or the like which is electrically connected to these electrodes.

Here, the container which contains the electrolyte fluid therein is divided by the partition wall to form the plurality of the cells. This partition wall has a continuous hole which makes each cell communicate with each other provided therein, and this continuous hole has the flow-channel opening/closing portion which opens/closes the flow channels between each cell provided therein.

When the electrolyte fluid is injected into the container, and when the electrolyte fluid is injected through the injection port, the flow channels between each cell are opened by the flow-channel opening/closing portion, and each cell communicates with each other. After the electrolyte fluid has been injected into the container, the flow channels between each cell are closed by the flow-channel opening/closing portion.

Thereby, the electrolyte fluid can be easily injected into the plurality of the cells by one injection operation, and also the electric charge is prevented from migrating between the cells after the electrolyte fluid has been injected. As a result, a desired voltage can be obtained.

In the above described second aspect, the flow-channel opening/closing portion may also be arranged on one straight line which passes the injection port and the continuous hole, and is an elastic body having a slit therein.

By being formed in this way, the flow-channel opening/closing portion makes a pipe through which the electrolyte fluid flows such as a syringe needle to penetrate through the injection port and the continuous hole, thereby enables the electrolyte fluid to be injected into the container from the pipe, also makes the pipe to expand the slit and open the flow channels between each cell, and enables the electrolyte fluid to be supplied into each cell. When this pipe is extracted from the injection port and the continuous hole, the slit is blocked due to an elastic force of the elastic body, the flow channels between each cell are closed, and the electric charge can be prevented from migrating between the cells.

In the above described second aspect, the flow-channel opening/closing portion may also be a valve which opens/closes the flow channels between each of the cells.

By being structured in this way, the valve opens and makes each cell communicate with each other when the electrolyte fluid is injected into the container, and the valve closes and can prohibit the communication between each cell after the electrolyte fluid has been injected into the container. Thereby, the electrolyte fluid can be easily injected into the plurality of the cells in one injection operation, and also the electric charge can be prevented from migrating between the cells after the electrolyte fluid has been injected.

The above described second aspect may include a plurality of the valves, and a connection mechanism which connects the plurality of the valves with each other.

By being structured in this way, the connection mechanism can simultaneously open/close the plurality of the valves, and the valves can be surely and easily opened/closed when the electrolyte fluid is injected into the container and after the electrolyte fluid has been injected into the container.

In the above described second aspect, the flow-channel opening/closing portion may also be a non-return valve which passes the electrolyte fluid in one direction from the cell to which the electrolyte fluid is injected, to the other cells. By being structured in this way, the battery can pass the electrolyte fluid to the other cells from the cell (injection cell) for which the electrolyte fluid is injected without needing the opening/closing operation of the valve or the like, and can prohibit the electrolyte fluid from flowing to the injection cell from another cell. Thereby, the electrolyte fluid can be easily injected into the plurality of the cells by one injection operation, and also the electric charge can be prevented from migrating between the cells.

In the above described second aspect, the partition wall may form a common flow channel which is adjacent to each of the cells, the injection port may be provided in the common flow channel, and the continuous hole and the flow-channel opening/closing portion may be provided in the partition wall which separates the common flow channel from each of the cells.

By being structured in this way, the flow-channel opening/closing portion opens the common flow channel and the flow channels between each cell, when the electrolyte fluid is supplied into the common flow channel through the injection port. After the electrolyte fluid has been injected into the common flow channel, the flow-channel opening/closing portion closes the common flow channel and the flow channels between each cell. Thereby, the electrolyte fluid can be easily injected into the plurality of the cells through the common flow channel in one injection operation, and also the electric charge can be prevented from migrating between the common flow channel and each cell and between the cells.

In the above described second aspect, each of the cells may also be arranged so as to be adjacent in the outside of the common flow channel.

By arranging the cells in this way, the battery can arrange many cells so as to be adjacent to one common flow channel, and can be down-sized.

In the above described second aspect, the battery may have a flow channel formed therein which makes the single injection port communicate with the plurality of the cells, on the condition that the continuous hole is opened by the flow-channel opening/closing portion.

In the above described second aspect, the battery may also have an electrical-connection switching portion provided therein which switches an electrical connection between the positive electrode and the negative electrode in each of the cells.

By being provided in this way, the electrical-connection switching portion can flexibly select and use the combination of parallel connection or serial connection of each cell.

The above described second aspect is means for connecting the cells of the fuel cell, and can be used for the fuel cell according to the first aspect.

A third aspect according to the present invention is an electrode for a fuel cell, which includes: a porous negative electrode that oxidizes a fuel; a positive electrode that reduces oxygen; and an ion-conducting membrane that interposes between the negative electrode and the positive electrode, wherein the negative electrode is arranged so as to have a gap between the negative electrode and the ion-conducting membrane.

There are cases which are supposed to use a cation permeable membrane and use an anion permeable membrane as the ion-conducting membrane. The former case is a case in which an electric power is generated by moving a proton that is a cation, through the membrane, and the latter case is a case in which the electric power is generated by moving a hydroxy ion that is an anion, through the membrane.

According to the third aspect according to the present invention, when the cation permeable membrane is used as the ion-conducting membrane, the fuel is oxidized in the negative electrode, and the electron and the proton are emitted from the fuel. On the other hand, oxygen is reduced by the emitted electron and proton on the positive electrode, and an electric current can be taken out by making the electron which moves from the negative electrode to the positive electrode pass through an external circuit. When a sugar solution is used as a fuel, a gap formed between the negative electrode and the ion-conducting membrane is also filled with the sugar solution. Accordingly, the proton which has been emitted in the negative electrode is transferred to the ion-conducting membrane from the filled sugar liquid, and is further transferred to the positive electrode.

In this case, the face on the ion-conducting membrane side of the negative electrode, which has been conventionally brought in close contact with the ion-conducting membrane, is also opened for the fuel, and the exposed surface area of the negative electrode increases, which is exposed to the fuel. Thereby, the fuel is promoted so as to diffuse and migrate in the negative electrode between the inside and the outside of the negative electrode, and a stable output current can be obtained while maintaining the power generation efficiency even when a sugar solution with comparatively high viscosity is used as the fuel.

Further, when the anion permeable membrane is used as the ion-conducting membrane, the fuel is oxidized in the negative electrode, and the electron and the proton are emitted from the fuel. On the other hand, the emitted electron and oxygen are reduced on the positive electrode together with water in the periphery to produce a hydroxy ion. An electric current can be taken out by making the electron which moves from the negative electrode to the positive electrode pass through the external circuit. When the sugar solution is used as the fuel, the gap formed between the negative electrode and the anion permeable membrane is also filled with the sugar solution. Accordingly, the proton which has been emitted in the negative electrode and the hydroxy ion which has been transferred to the filled sugar liquid from the positive electrode through the anion permeable membrane form water.

In other words, the difference of whether the cation permeable membrane is used or the anion permeable membrane is used is that the electric power is generated by transferring the proton between the electrodes or the electric power is generated by transferring the hydroxy ion, and both cases have a structure of using the ion-conducting membrane.

The third aspect is the invention relating to the electrode to be set in an inner part of the fuel cell, and it is obvious that the electrode is used for the fuel cell according to the first aspect.

A fourth aspect according to the present invention is an electrode for a fuel cell, which includes: a porous negative electrode that oxidizes a fuel; a positive electrode that reduces oxygen; and an ion-conducting membrane that interposes between the negative electrode and the positive electrode, wherein the negative electrode has asperity formed on a surface thereof.

According to the fourth aspect according to the present invention, the asperity formed on the surface of the negative electrode increases the exposed surface area of the negative electrode to the fuel, thereby the fuel is promoted so as to diffuse and migrate in the negative electrode between the inside and the outside of the negative electrode, and a stable output current can be obtained while maintaining the power generation efficiency even when the sugar solution is used as the fuel.

In the above described fourth aspect, the negative electrode may also have the surface formed into a fin shape, and may also have a groove formed on the surface.

By being structured in this way, the negative electrode can effectively increase the exposed surface area to the fuel.

In the above described fourth aspect, the negative electrode may also be arranged so as to have a gap between the negative electrode and the ion-conducting membrane.

A fifth aspect according to the present invention is a fuel cell provided with the electrode for the fuel cell, which is described in any one of the third aspect and the fourth aspect.

{Advantageous Effects of Invention}

According to the first aspect according to the present invention, the fuel cell shows such an effect that the fuel cell can be operated for a long time while reducing the size in order to be implanted in the living body.

According to the second aspect according to the present invention, the battery shows such an effect that a fuel liquid can be easily injected into the plurality of the cells, and the electric charge can be prevented from migrating between the cells.

According to the third to the fifth aspects of the present invention, the electrode shows such an effect that the fuel cell can obtain the stable output current while maintaining the power generation efficiency even when the sugar solution is used as the fuel.

{Brief Description of Drawings}

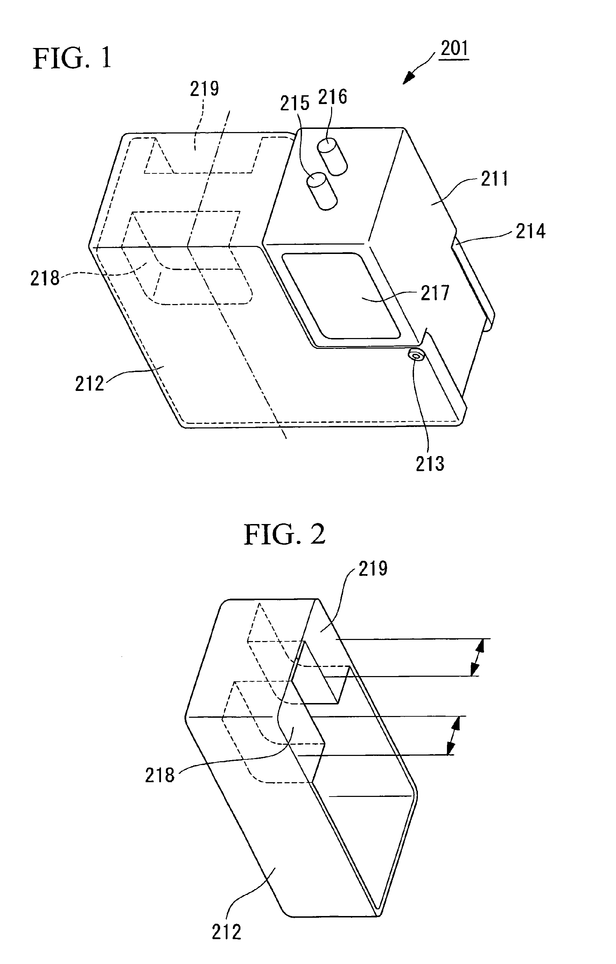

{Fig. 1}
Fig. 1 is a whole schematic diagram of a fuel cell according to a first embodiment of the present invention.
{Fig. 2}
Fig. 2 is a sectional view of a fuel bag in the cross section shown by a dashed line of Fig. 1.
{Fig. 3}
Fig. 3 is an assembly drawing illustrating a structure of an electrode.
{Fig. 4}
Fig. 4 is a longitudinal sectional view of the electrode of Fig. 3.
{Fig. 5}
Fig. 5 is a partially enlarged view of the electrode of Fig. 4.
{Fig. 6}
Fig. 6 is a model view illustrating an oxidation-reduction reaction in each electrode.
{Fig. 7}
Fig. 7 is a whole schematic diagram of a fuel cell according to a second embodiment of the present invention.
{Fig. 8}

Fig. 8 is a perspective view of a fuel bag of Fig. 7.

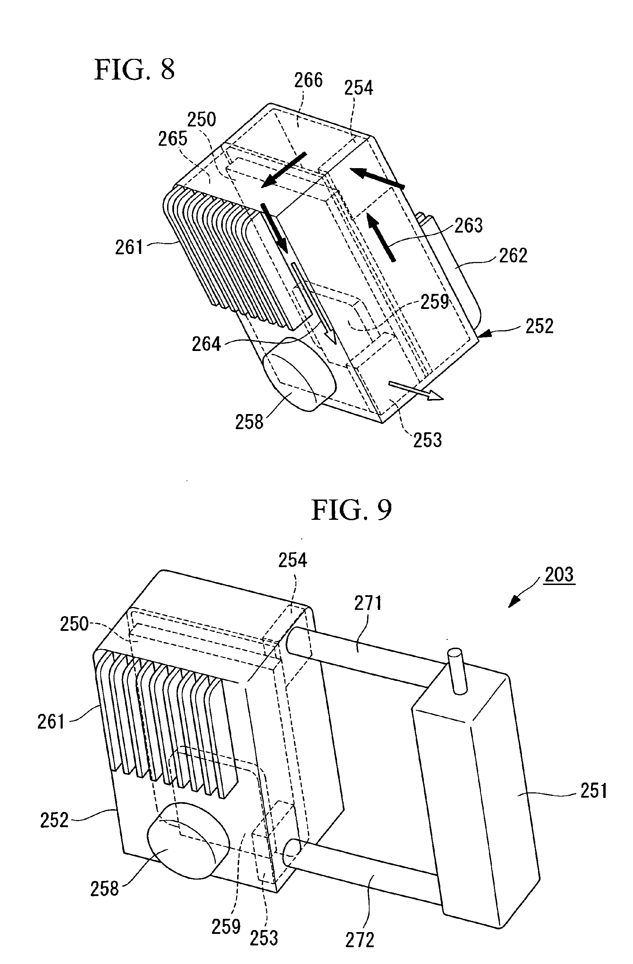

{Fig. 9}

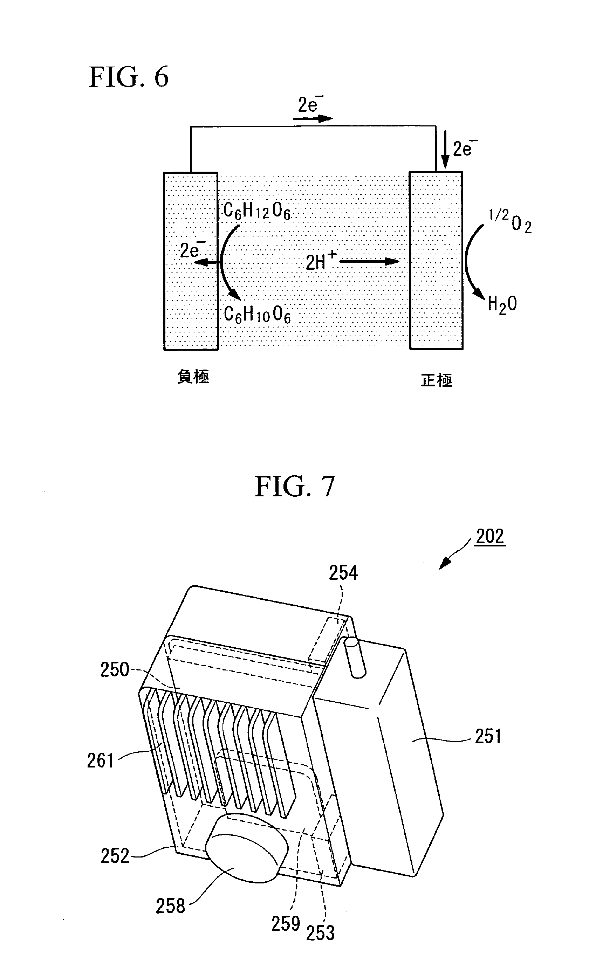

Fig. 9 is a whole schematic diagram illustrating a modified example of the fuel cell of Fig. 7.

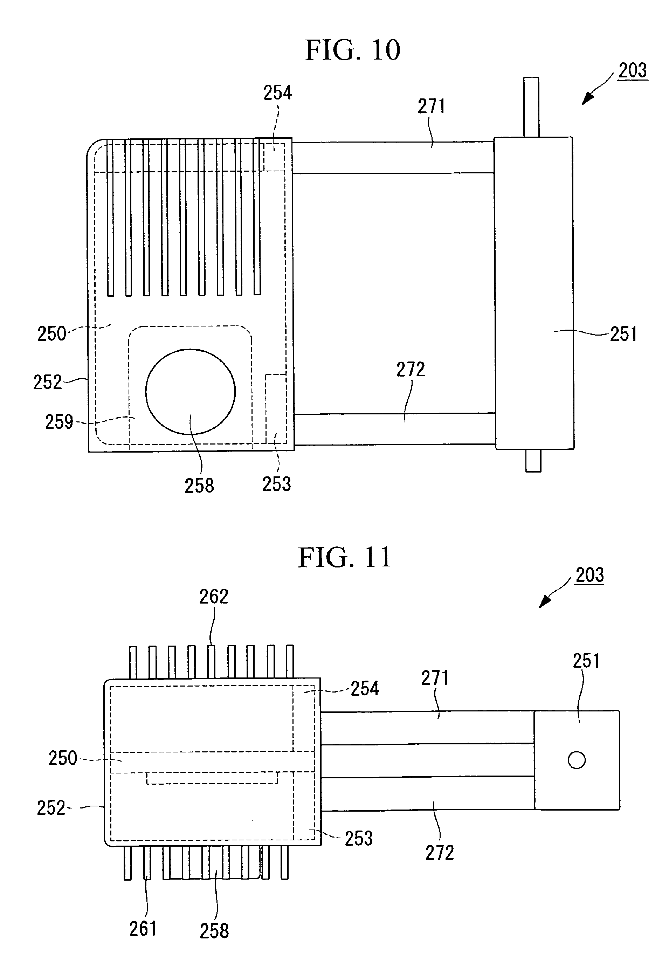

{Fig. 10}

Fig. 10 is a front view of the fuel cell of Fig. 9.

{Fig. 11}

Fig. 11 is a top view of the fuel cell of Fig. 9.

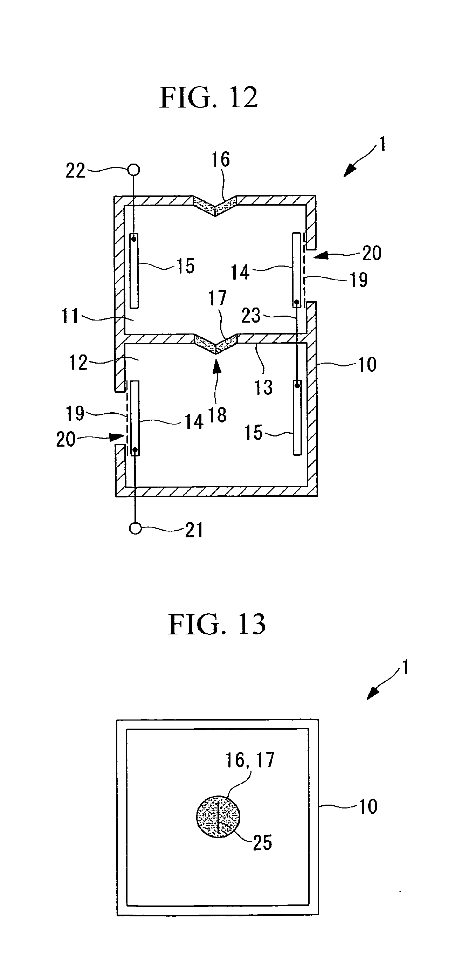

{Fig. 12}

Fig. 12 is a longitudinal sectional view illustrating a schematic structure of a battery according to a third embodiment of the present invention.

{Fig. 13}

Fig. 13 is a plan view of the battery of Fig. 12.

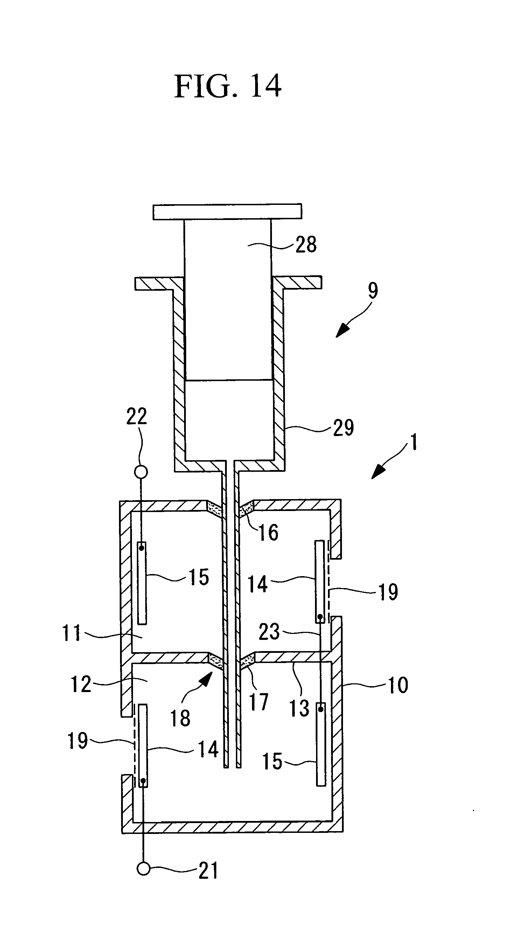

{Fig. 14}

Fig. 14 is a longitudinal sectional view when a syringe has been inserted into the battery of Fig. 12.

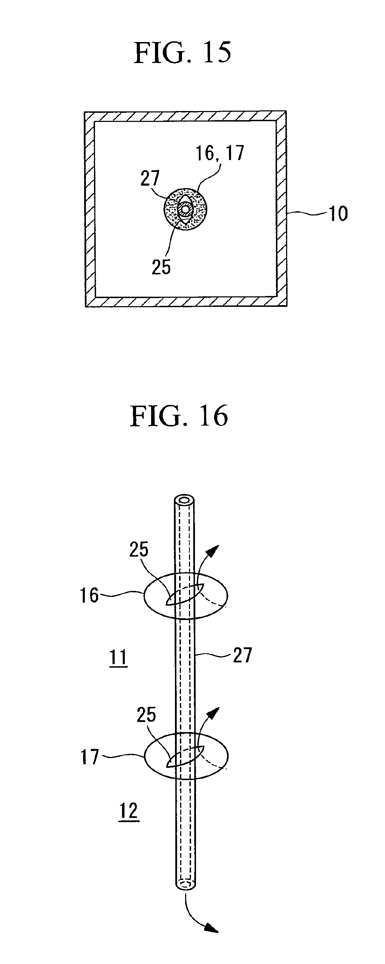

{Fig. 15}

Fig. 15 is a plan view of the battery of Fig. 14.

{Fig. 16}

Fig. 16 is a schematic view for describing a state of a syringe needle and a slit valve of Fig. 14.

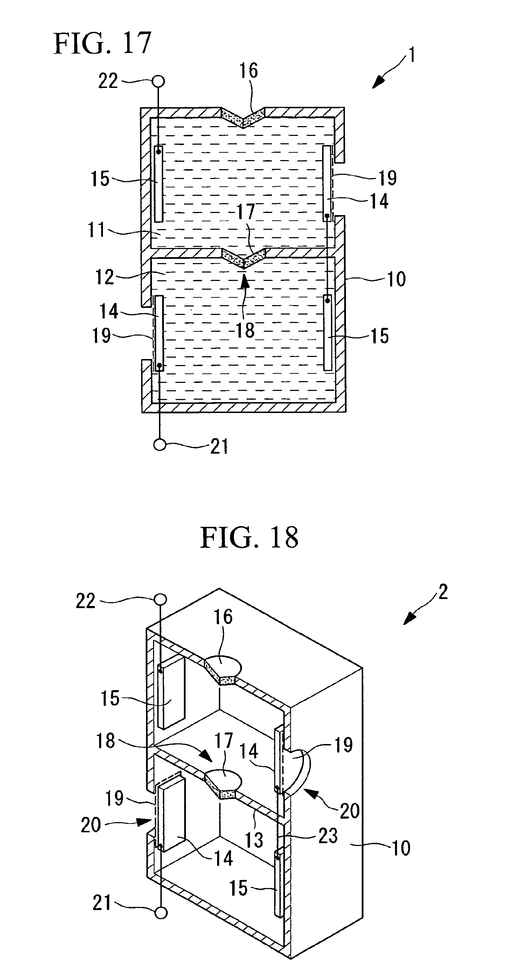

{Fig. 17}

Fig. 17 is a longitudinal sectional view illustrating a state in which an electrolyte solution has been injected into the battery of Fig. 12.

{Fig. 18 }

Fig. 18 is a longitudinal sectional view illustrating a schematic structure of a battery according to a fourth embodiment of the present invention.

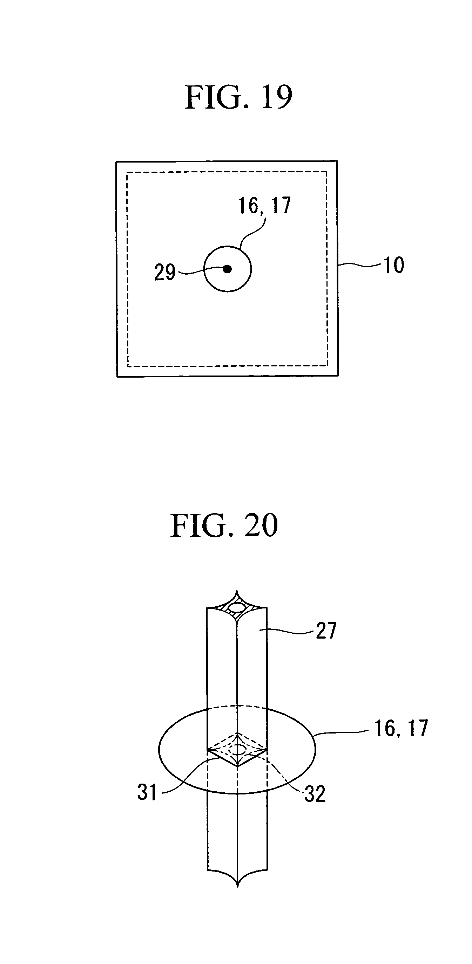

{Fig. 19}

Fig. 19 is a plan view of the battery of Fig. 18.

{Fig. 20}

Fig. 20 is a schematic view for describing a state when a syringe has been inserted into the battery of Fig. 18.

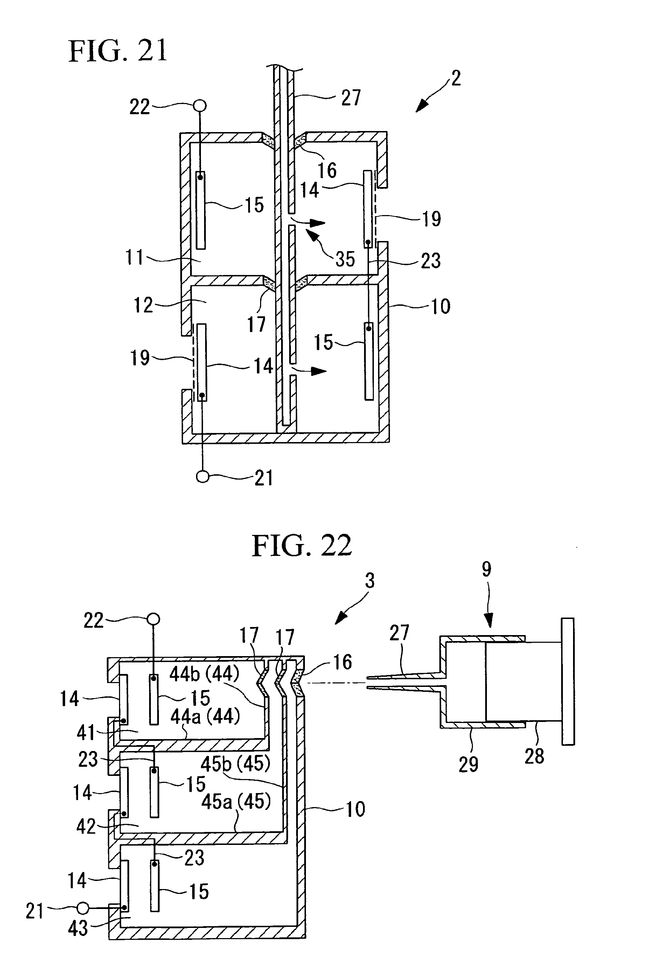

{Fig. 21}

Fig. 21 is a longitudinal sectional view illustrating the schematic structure of a modified example of Fig. 18.

{Fig. 22 }

Fig. 22 is a longitudinal sectional view illustrating a schematic structure of a battery according to a fifth embodiment of the present invention.

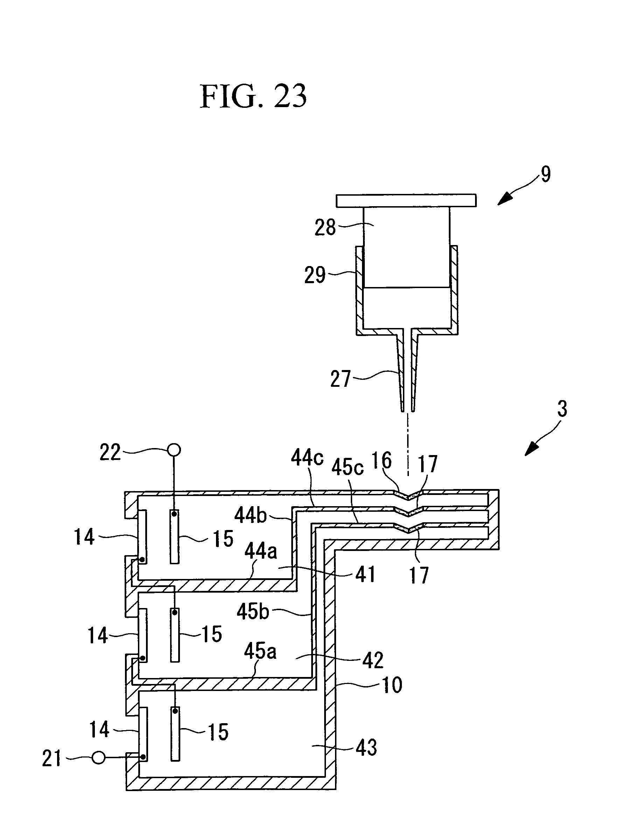

{Fig. 23}

Fig. 23 is a longitudinal sectional view illustrating the schematic structure of a modified example of Fig. 22.

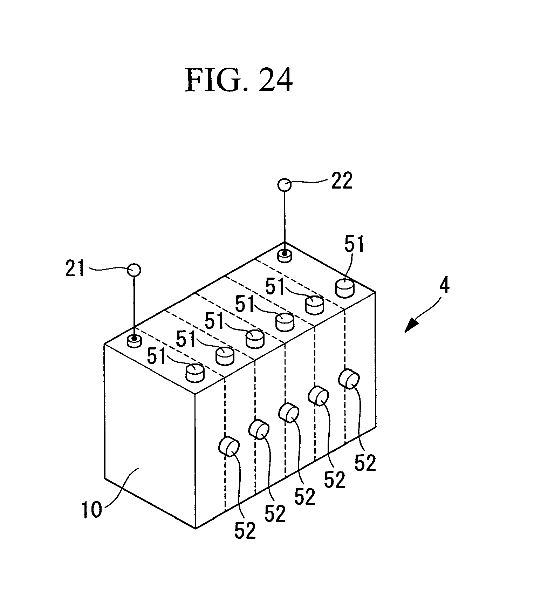

{Fig. 24}

Fig. 24 is a perspective view illustrating a schematic structure of a battery according to a sixth embodiment of the present invention.

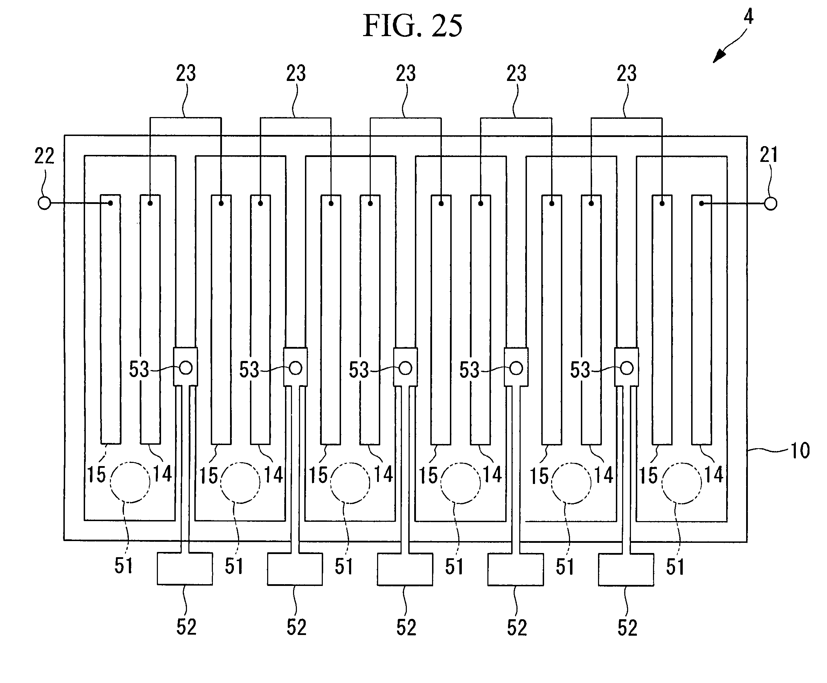

{Fig. 25}

Fig. 25 is a transverse sectional view of the battery of Fig. 24.

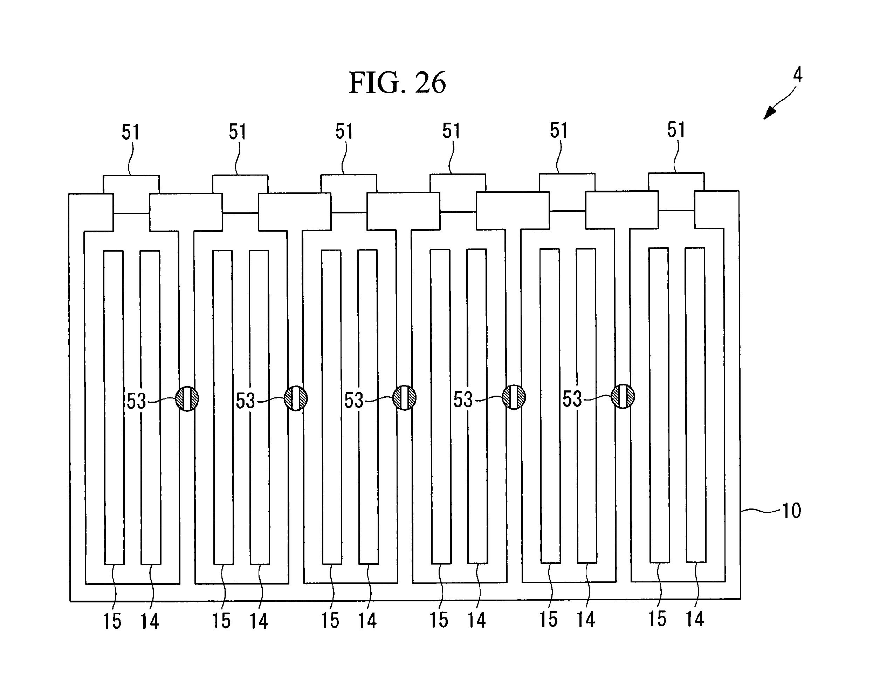

{Fig. 26}

Fig. 26 is a longitudinal sectional view of the battery of Fig. 24, and is a view for describing a state in which a manual valve has been closed.

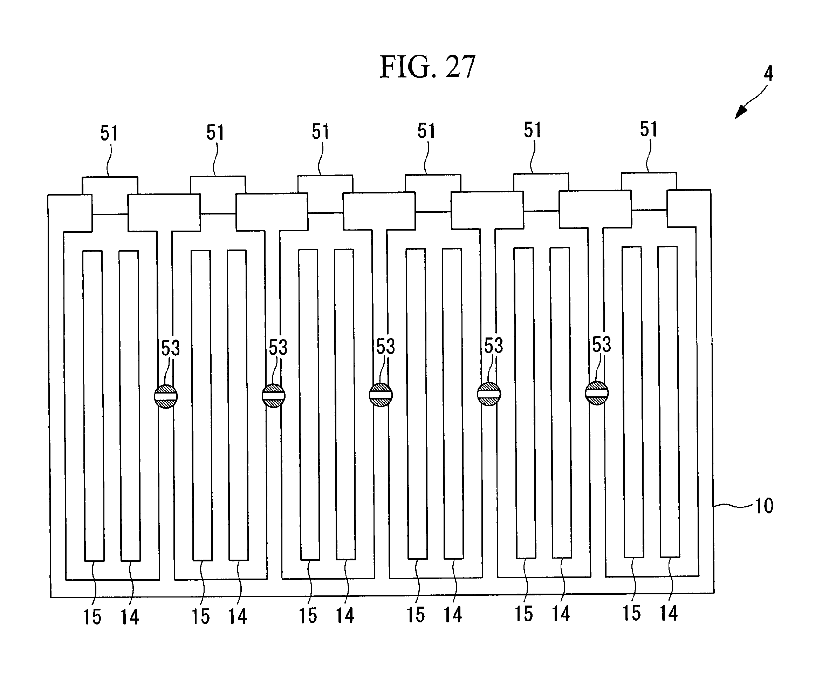

{Fig. 27}

Fig. 27 is a longitudinal sectional view of the battery of Fig. 24, and is a view for describing the state in which a manual valve has been opened.

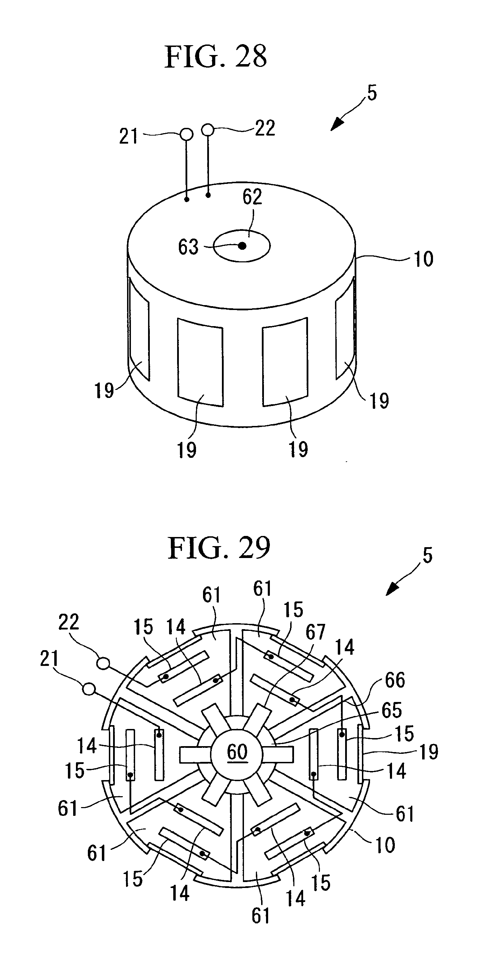

{Fig. 28}

Fig. 28 is a perspective view illustrating a schematic structure of a battery according to a seventh embodiment of the present invention.

{Fig. 29}

Fig. 29 is a transverse sectional view of the battery of Fig. 28.

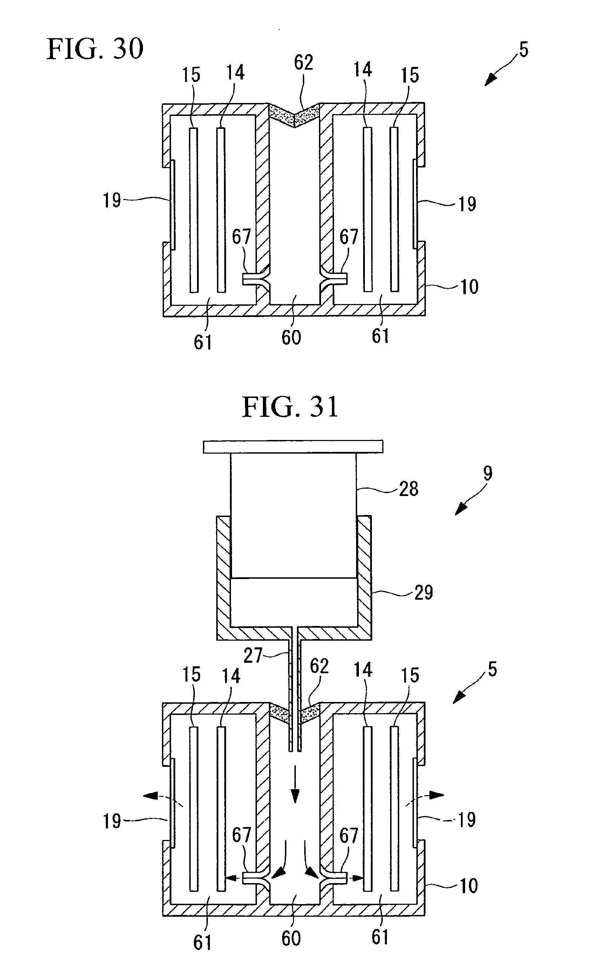

{Fig. 30}

Fig. 30 is a longitudinal sectional view of the battery of Fig. 28.

{Fig. 31}

Fig. 31 is a longitudinal sectional view for describing a state when a syringe has been inserted into the battery of Fig. 28.

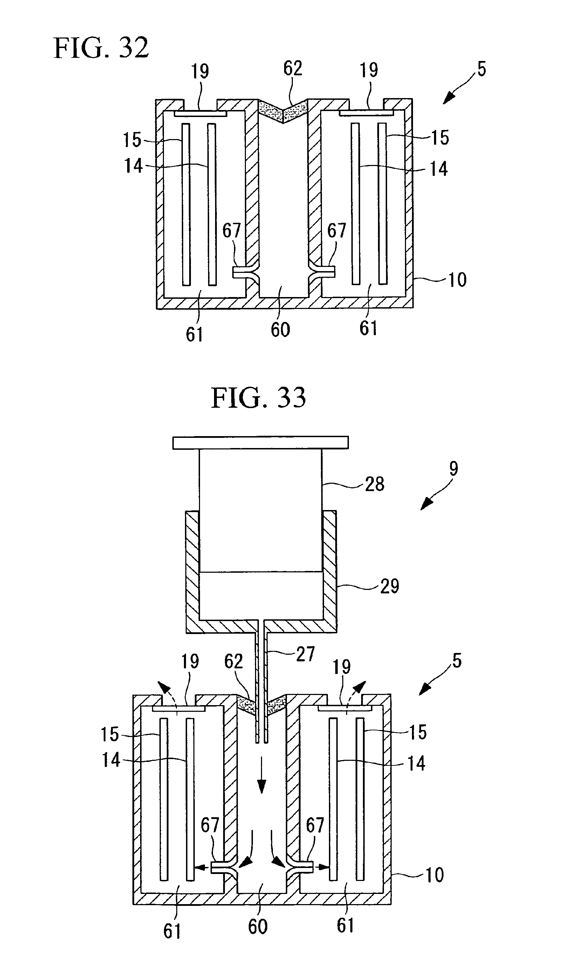

{Fig. 32}

Fig. 32 is a longitudinal sectional view illustrating the schematic structure of a modified example of Fig. 28.

{Fig. 33}

Fig. 33 is a longitudinal sectional view for describing the state when a syringe has been inserted into the battery of Fig. 32.

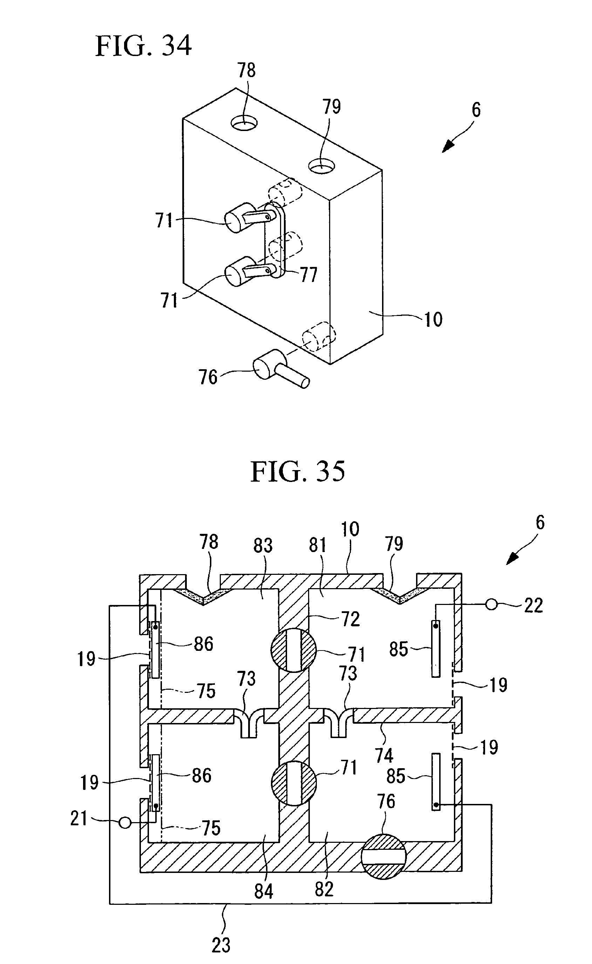

{Fig. 34}

Fig. 34 is a perspective view illustrating a schematic structure of a battery according to an eighth embodiment of the present invention.

{Fig. 35}

Fig. 35 is a longitudinal sectional view of the battery of Fig. 34.

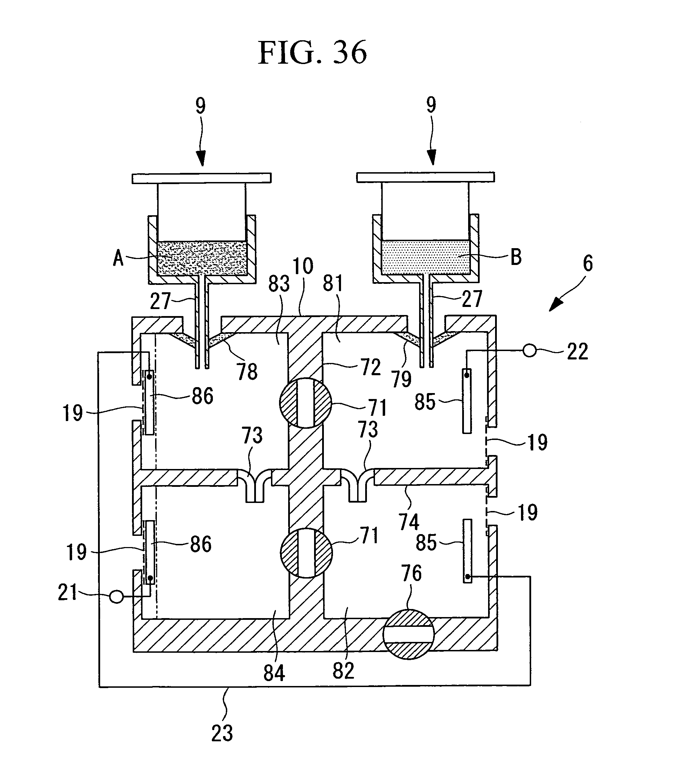

{Fig. 36}

Fig. 36 is a longitudinal sectional view for describing a state when a syringe has been inserted into the battery of Fig. 34.

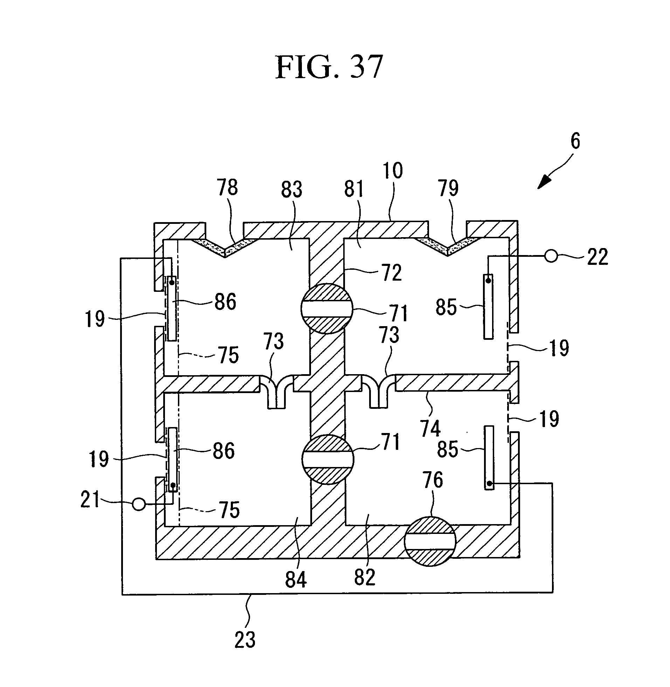

{Fig. 37}

Fig. 37 is a longitudinal sectional view for describing a state in which a manual valve of the battery of Fig. 34 has been opened.

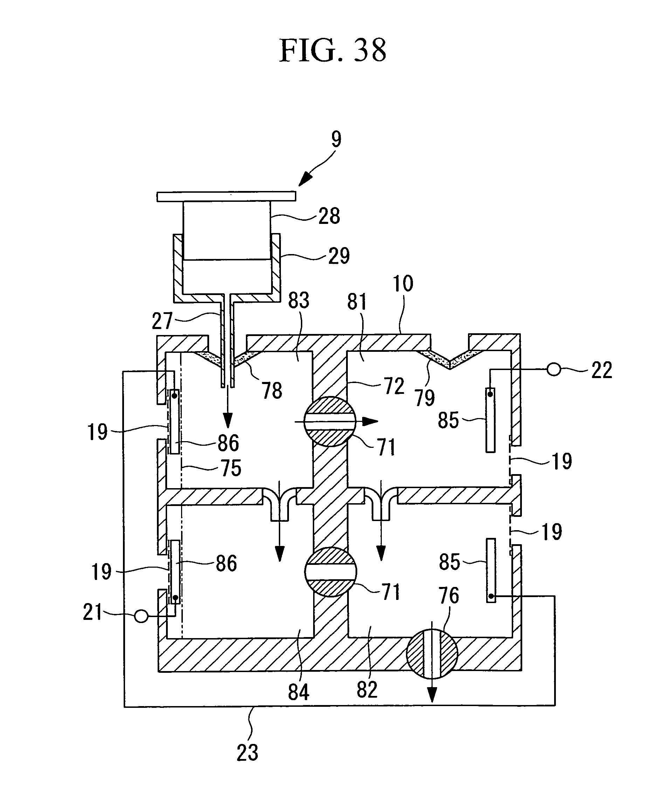

{Fig. 38 }

Fig. 38 is a longitudinal sectional view for describing a state when an electrolyte solution of the battery of Fig. 34 is discharged.

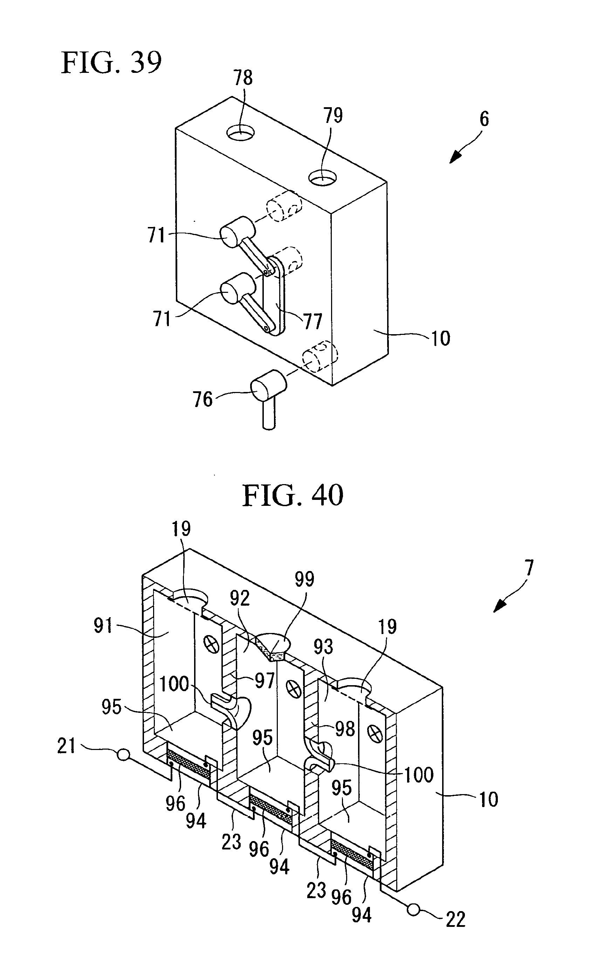

{Fig. 39}

Fig. 39 is a perspective view for describing the state when the electrolyte solution of the battery of Fig. 34 is discharged.

{Fig. 40}

Fig. 40 is a longitudinal sectional view illustrating a schematic structure of a battery according to a ninth embodiment of the present invention.

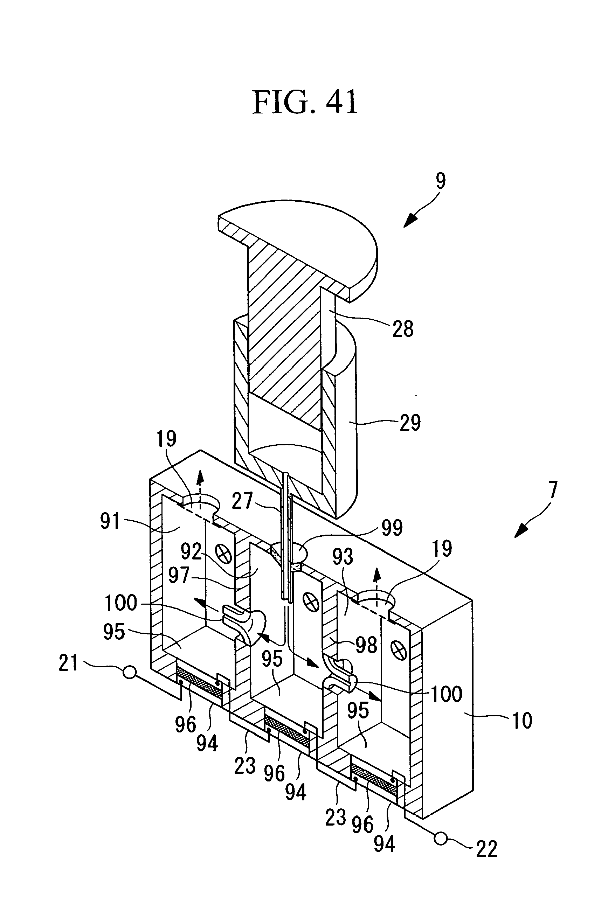

{Fig. 41}

Fig. 41 is a longitudinal sectional view for describing a state when a syringe has been inserted into the battery of Fig. 40.

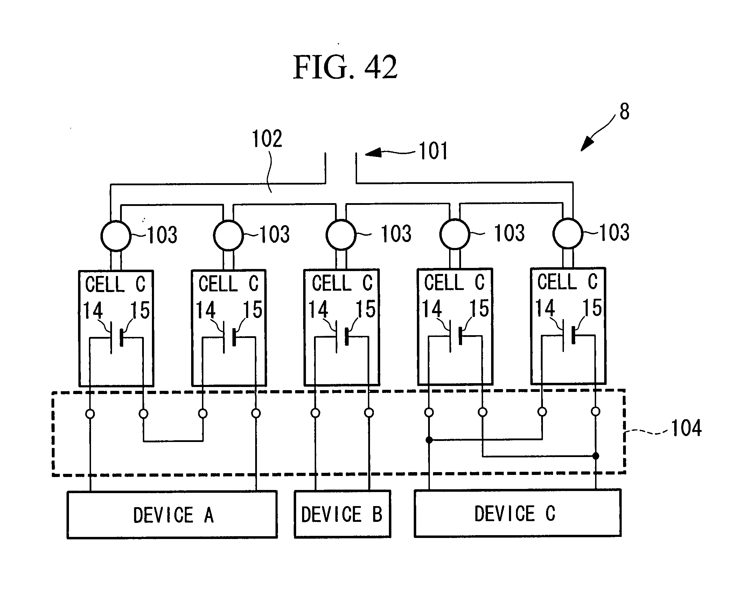

{Fig. 42}

Fig. 42 is a schematic view illustrating a schematic structure of a battery according to a tenth embodiment of the present invention.

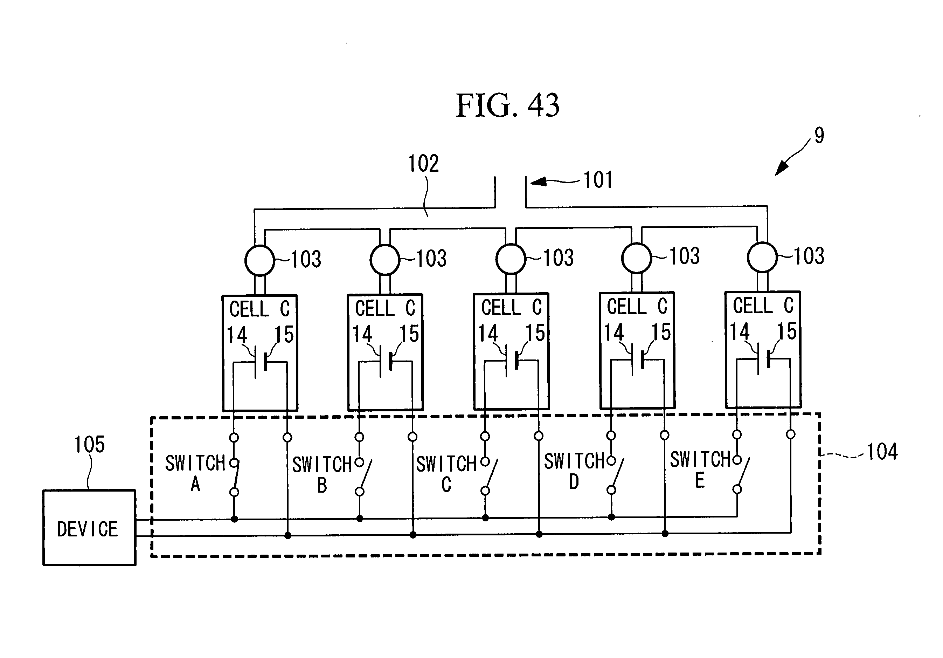

{Fig. 43}

Fig. 43 is a schematic view illustrating a schematic structure of a battery according to an eleventh embodiment of the present invention.

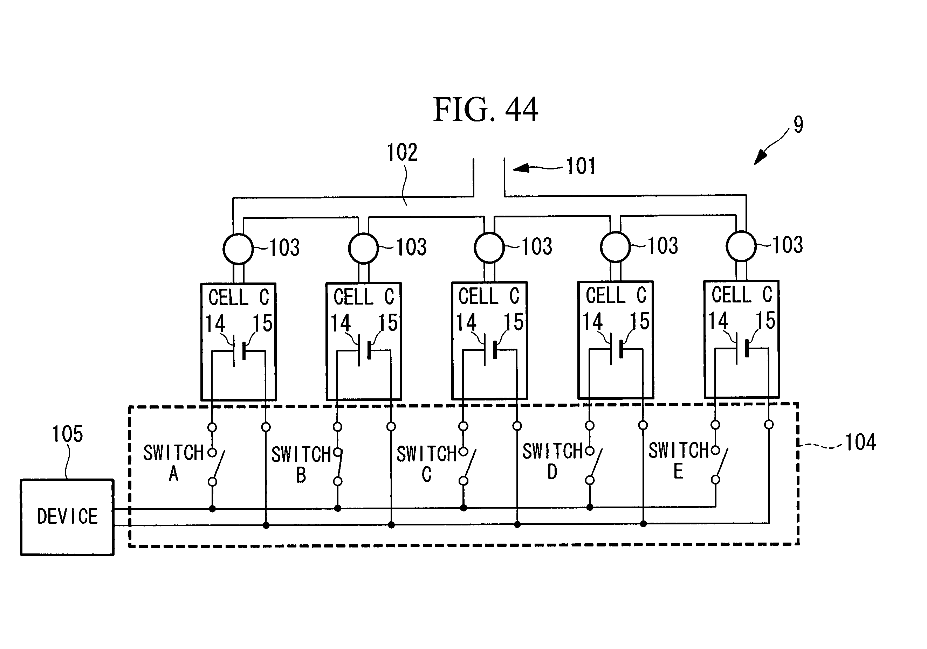

{Fig. 44}

Fig. 44 is a schematic view describing a state in which a cell for supplying an electric power has been switched in the battery of Fig. 43.

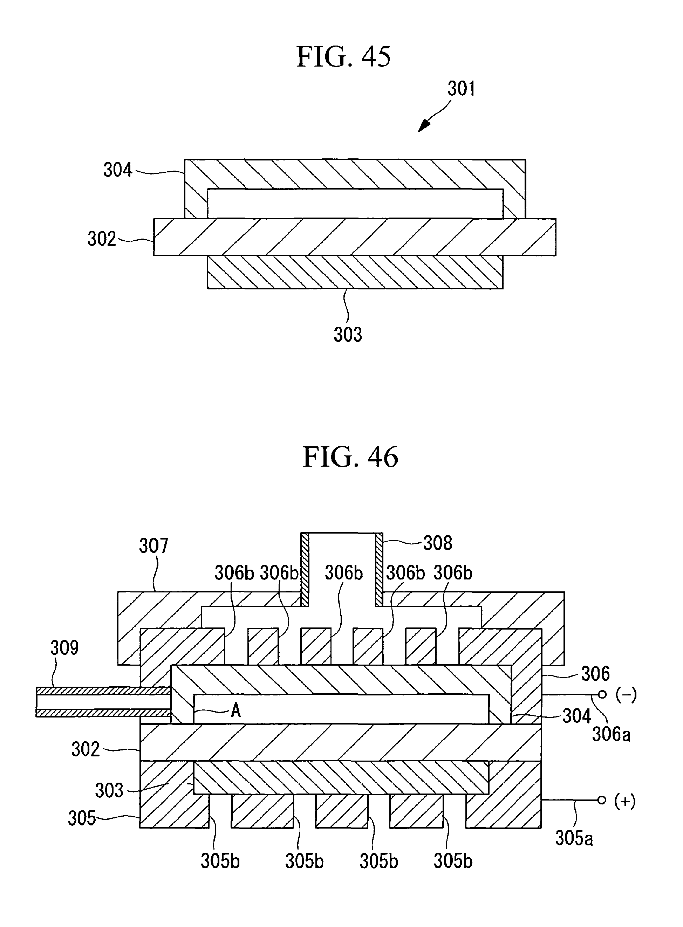

{Fig. 45}

Fig. 45 is a whole schematic diagram of an electrode for a fuel cell according to a twelfth embodiment of the present invention.

{Fig. 46}

Fig. 46 is a whole schematic diagram of a fuel cell using the electrode for a fuel cell of Fig. 45.

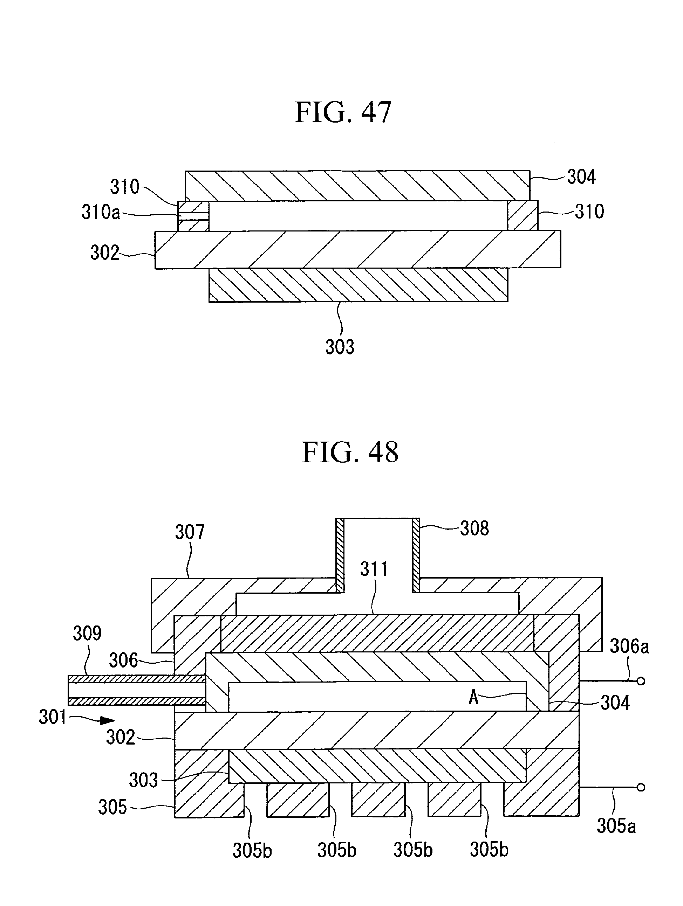

{Fig. 47}

Fig. 47 is a view illustrating a modified example of the electrode for a fuel cell of Fig. 45.

{Fig. 48}

Fig. 48 is a view illustrating a modified example of the fuel cell of Fig. 46.

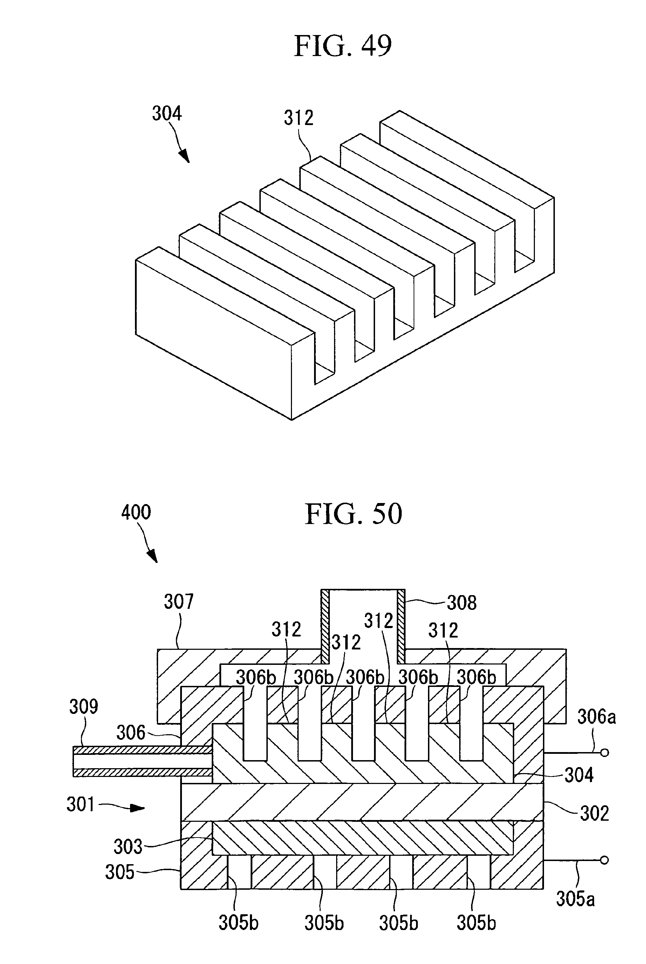

{Fig. 49}

Fig. 49 is a whole schematic diagram of an electrode for a fuel cell according to a thirteenth embodiment of the present invention.

{Fig. 50}

Fig. 50 is a whole schematic diagram of a fuel cell using the electrode for a fuel cell of Fig. 49.

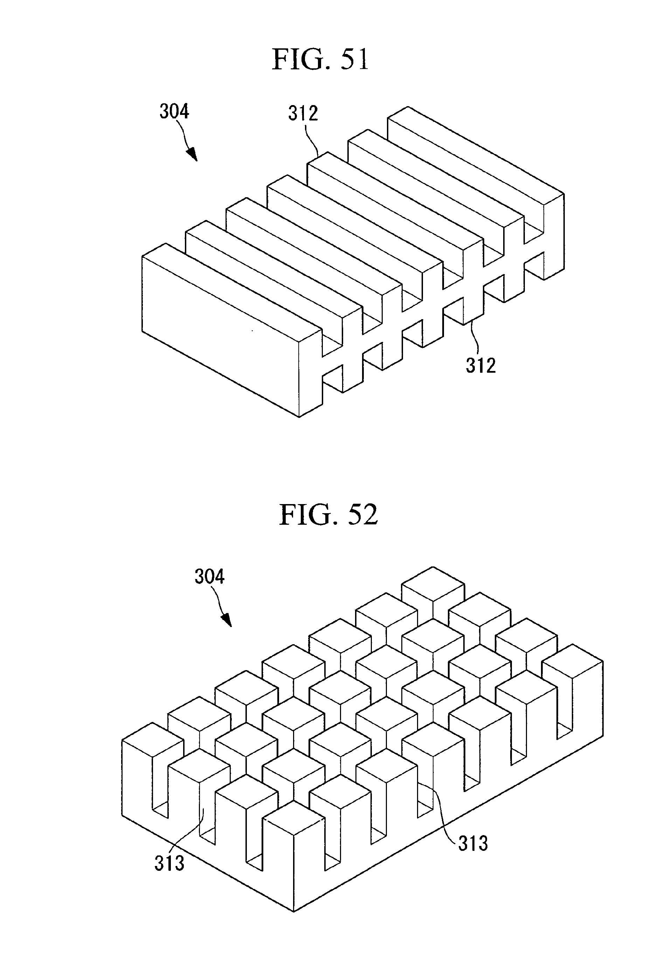

{Fig. 51}

Fig. 51 is a view illustrating a modified example of the electrode for a fuel cell of Fig. 49.

{Fig. 52}

Fig. 52 is a view illustrating another modified example of the electrode for a fuel cell of Fig. 49.

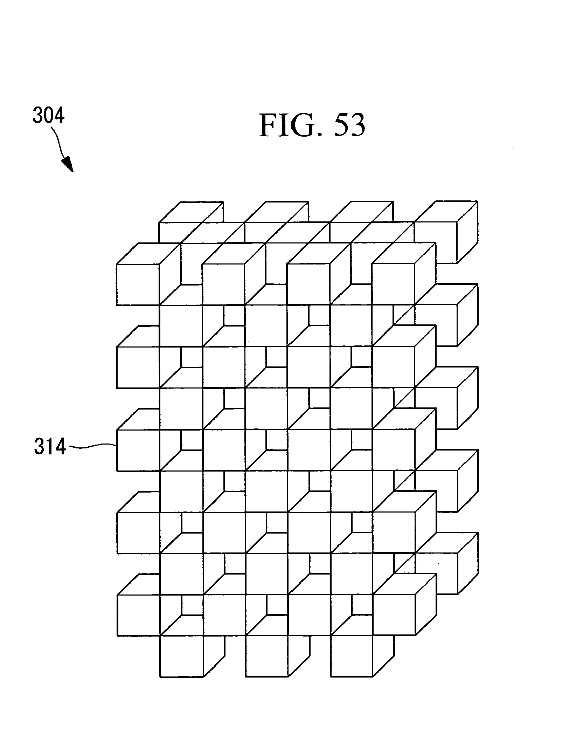

{Fig. 53}

Fig. 53 is a view illustrating another modified example of the electrode for a fuel cell of Fig. 49.

{Description of Embodiments}

[First embodiment]

A fuel cell according to a first embodiment of the present invention will be described below with reference to the drawings.

Firstly, circumstances under which the present inventors have extensively investigated a structure of the fuel cell according to the present invention will be described below.

The oxidation of sugar using a metal and a fuel cell using the oxidation of the sugar are reported in many patent literatures and research papers. For instance, the Publication of Japanese Patent No. 3518461 is known as a patent literature, and literatures described in the following (1) to (4) are known as non-patent literatures.

(1) J. Electroanal. Gem., "Concentration dependence of the mechanism of glucose oxidation at gold electrodes in alkaline media", 262, 1989, pp167-182
(2) "Preparation of gold electrode modified with pyridyl phosphonate and glucose oxidation capability thereof", proceedings in Meeting of West Japan Branch of the Chemical Society of Japan, 2004, page 74,
(3) "Preparation of electrode with function of glucose oxidation catalyst using gold nano-particle and development of fuel cell" and "Preparation of electrode having glucose oxidation capability and application thereof to glucose fuel cell", Abstracts in Meeting of the Electrochemical Society of Japan, 2005, page 212
(4) "Practical Bioelectrochemistry, glucose-air fuel cell", CMC Publishing Co., Ltd., March, 2007

Any fuel cell described in these literatures oxidizes glucose which becomes a fuel, by using a noble metal such as gold, silver and platinum as a catalyst, as described above.

Non-Patent Literature (1) describes the details of a mechanism of electron transfer occurring in sugar, particularly, glucose when gold of a noble metal is used, in other words, a mechanism of taking out an electron from the glucose, and describes that the oxidation of the glucose spreads while initiating at a hydroxyl group which has adsorbed to the gold. Similarly, the Publication of Japanese Patent No. 3518461 also describes a mechanism by which an electron that has been emitted by the oxidation of the sugar is transferred via a chain body through a hydroxyl group.

These mechanisms use epimerization of sugar, which is a reaction that the absolute arrangement of asymmetric carbon loses a proton and re-protonation occurs in the same side, whereby a conversion reaction of the sugar progresses in an alkaline solution more quickly than in an acidic solution. For this reason, many hydroxyl groups existing in the alkaline solution work as a trigger of the sugar oxidation, and the hydroxyl group which easily adsorbs to gold simultaneously works as an antenna of the electron transfer and results in helping the electron to be transferred to the gold. By this mechanism, the oxidation of glucose is promoted by the existence of the hydroxyl group and a noble metal, particularly the gold, and an electric power is generated.

In addition, because the hydroxyl group is important for the oxidation of sugar on the noble metal, an alkaline environment becomes optimal. However, the alkaline environment is effective for enhancing the electric power generation capability, but is not indispensable. For instance, in the above described Non-Patent Literatures (2) and (3), high electric power generation capability is obtained in a neutral aqueous solution of glucose as well, by using gold and platinum. As described in the above described Non-Patent Literatures (2) and (3), the catalytic ability of platinum, silver or the like itself for the sugar oxidation is inferior to that of gold, but the platinum, silver or the like shows a high performance as a co-catalyst by being used together with the gold, and does not necessarily need alkalinity though depending on the combinations or structures of the noble metals.

In other words, in order to take the electron generated from the glucose which is the sugar by oxidation out to the electrode, such a technique may be conducted as to reduce an energy threshold so that the electron transfer easily occurs, in addition to using the existence of the hydroxyl group. It can be assumed that the above described use of gold and platinum enhances an electric field of the surface due to the combination of the mutual noble metals, and has succeeded in transferring an electron from the glucose.

From the above description, it is understood that an environment which has hydroxyl groups so as to present alkalinity is not necessarily indispensable by devising an electrode structure formed by combining the noble metals.

In addition, it is known that the standard oxidation-reduction potential of the noble metal is positive. In other words, it is known that a very positive potential with respect to the oxidation-reduction potential of hydrogen is necessary for oxidizing the noble metals in the natural world, and the noble metals cannot be easily oxidized. As for the gold, for instance, a potential more positive than the reference potential of hydrogen by +1.52 V is necessary for oxidizing gold into its trivalent ion. In addition, in order to oxidize the gold into a monovalent ion, a further positive potential is necessary and the potential reaches +1.83 V. This fact suggests that it is difficult to occur that gold is oxidized and the activity is lowered in an aqueous solution having a decomposition voltage of 1.5 V, in a fuel cell which uses an aqueous solution as a fuel.

For this reason, the fuel cell 201 according to the present embodiment uses a noble metal having such characteristics as an oxidation electrode of sugar. Thereby, an extremely stable activity is kept for a long time, and an electric power can be generated for a long period. In addition, the electrode shall be used as an oxidation electrode for oxidizing sugar that is a fuel, which uses fine particles of a noble metal, particularly, fine particles with a nanometer size (hereinafter referred to as "nano-particle") instead of using such an enzyme as described in Patent Literature 1.

Next, the structure of the fuel cell 201 according to the present embodiment will be described below. As illustrated in Fig. 1, the fuel cell 201 according to the present embodiment is a fuel cell 201 which is implanted in the living body, and includes a container 211 that contains an electrolyte solution therein and a fuel bag 212 (storage portion) having a sugar fuel such as glucose stored therein. The container 211 and the fuel bag 212 are mutually connected to each other through an injection port 213 and a discharge port 214.

The container 211 is, for instance, a closed container constituted by a material having biocompatibility such as titanium, and is structured so as to contain the electrolyte solution in the inner part. The container 211 has a pair of electrodes provided in the inner part, which have a noble metal fixed on the surface. Output terminals 215 and 216 for outputting the electric power to equipment such as a pacemaker are formed on ends of the pair of the electrodes. An aeration portion 217 having air permeability and waterproofness is formed on one part of the outer surface of the container 211. The aeration portion 217 is constituted, for instance, by a carbon fluoride resin such as polytetrafluoro-ethylene (ethylene tetrafluoride).

The pair of the electrodes are constituted by an anode and a cathode which are two types of electrodes having a catalyst of a noble metal carried thereon. The anode works as a negative electrode of the fuel cell 201, on which the sugar is oxidized, and the cathode works as a positive electrode of the fuel cell 201, on which oxygen is reduced.

The anode and the cathode each having the catalyst of the noble metal carried thereon are set in the inner part of the container 211, and are immersed in the electrolyte solution containing the sugar fuel. The anode and the cathode are connected to respective output terminals. Each output terminal is structured so as to be connected to medical equipment such as a pacemaker which is implanted in the living body, and so as to supply the electric power to the medical equipment.

Here, the detailed structure of these anode and cathode will be described below.

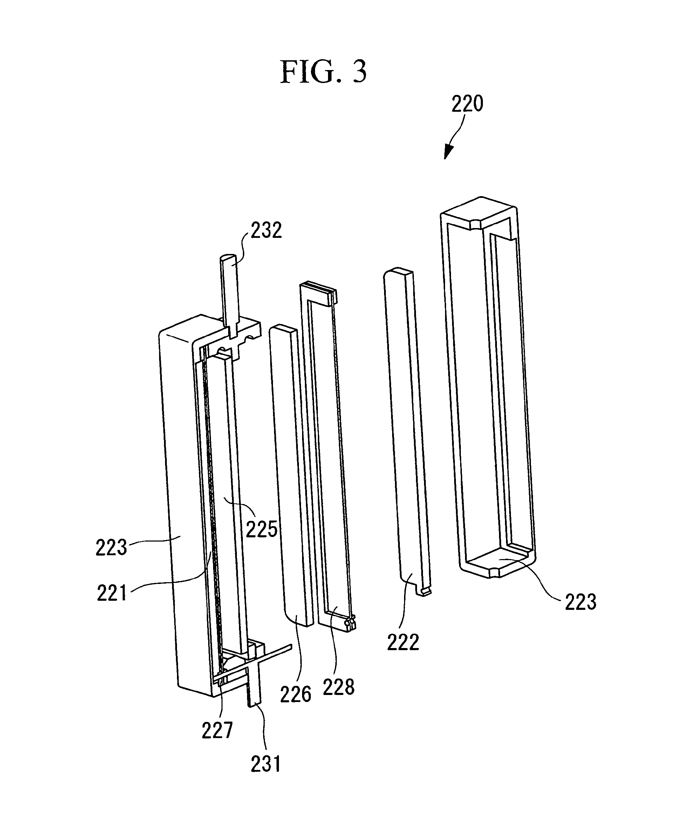

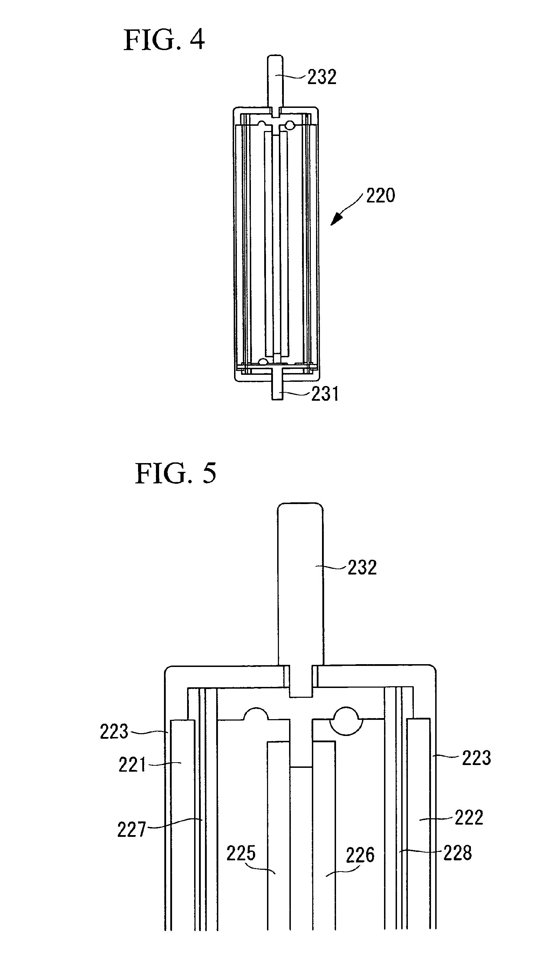

Fig. 3 to Fig. 5 illustrate the structure of an electrode 220 arranged in the inner part of the container 211. Here, the structure is illustrated as one example, in which electrodes that are two sheets each and four sheets in total are equipped and are connected in parallel. A plus terminal 231 and a minus terminal 232 are formed in both ends of the electrode 220.

As illustrated in Fig. 3 to Fig. 5, the cathodes 221 and 222 are inserted in a case 223 which is formed so as to be extremely thin. The case 223 is constituted by a material having air permeability such as polytetrafluoro-ethylene because of needing to come in contact with the living body and take oxygen dissolved in the living body into the inner part of the container 211.

The polytetrafluoro-ethylene has gas permeability because of having a pore structure, and is known to have adequate oxygen permeability as is used for an oxygen enrichment membrane, for instance. In addition, the polytetrafluoro-ethylene has also biocompatibility, and can take the dissolved oxygen into the inner part of the container 211 by using its oxygen permeability. Thereby, the case 223 can take oxygen from the outside of the case 223 to the cathodes 221 and 222 arranged so as to be adjacent to the case.

A hydrogen ion may be exchanged between the anodes 225 and 226 and the cathodes 221 and 222. Particularly, the cathodes 221 and 222 do not need sugar. Accordingly, if there are hydrogen permeable membranes 227 and 228, the membranes serve more effectively for preventing the extra oxidation of sugar.
The hydrogen permeable membranes 227 and 228 are installed so as to suppress the loss caused by a crossover phenomenon which originates in the oxidation of sugar occurring on the above described cathodes 221 and 222, and are not actually indispensable because the electric power can be generated even when the membranes are not installed. Particularly, in the fuel cell for being implanted in the living body, which is operated at a thrifty electric power, the occurrence of some crossover phenomenon does not cause a large problem.

However, when the hydrogen permeable membranes 227 and 228 are arranged, the sugar fuel which circulates and convects shall be supplied between the hydrogen permeable membranes 227 and 228 which oppose the anodes 225 and 226.
At this time, only a supporting electrolyte may be previously injected into a gap between the hydrogen permeable membranes 227 and 228 and the cathodes 221 and 222, when the battery is assembled.

As described above, the noble metal to be used on the anodes 225 and 226 and the cathodes 221 and 222 is preferably a nano-particle of the noble metal using gold and/or platinum, and it is considered to use each noble metal singly or use the noble metals by mixing a plurality of the noble metals. Particularly, the nano-particles of the gold are most effective for the anodes 225 and 226 because the sugar is oxidized thereon. On the contrary, the nano-particles of the platinum are most effective for the cathodes 221 and 222 because oxygen is reduced thereon.

In addition, it is also considered to use silver, iridium, osmium or ruthenium which works as a co-catalyst, together with the nano-particle of a noble metal. In order to more efficiently oxidize sugar and reduce oxygen as described above, it is important to enhance an electric field of the nano-particles which work as a catalyst and to lower a threshold of the electron transfer, and it is effective to combine a noble metal with a metal which becomes a co-catalyst.

Incidentally, a parallel structure of the electrodes is not indispensable, but is a structure for obtaining the area by arranging the cathodes 221 and 222 in both faces of the case 223, in order to take oxygen which dissolved in the living body at a low concentration into the container as effectively as possible. Accordingly, if the electric power or oxygen is sufficient, the structure is not necessarily limited to the above described parallel structure. In addition, the hydrogen permeable membranes 227 and 228 are arranged between both electrodes, but the arrangement is not indispensable.

As illustrated in Fig. 2, the fuel bag 212 is constituted by a material having biocompatibility, for instance, such as silicon rubber and polyurethane rubber, and is structured so as to store a sugar fuel such as glucose in the inner part thereof. The fuel bag 212 has septa (injection/discharge port) 218 and 219 provided therein which are formed so as to have a thick wall. These septa 218 and 219 are formed so as to be freely penetrated by a syringe needle, and function as an injection/discharge port for injecting the fuel from the outside into the container 211 or discharging it from the container 211.

As illustrated in Fig. 2, the septa 218 and 219 are one part of the fuel bag 212, and are structured so as not to be ruptured even when a needle of an injector is inserted thereinto, because only the portion is formed so as to form a thick wall. In addition, if the needle of the injector is supposed to be inserted into the septum 218 side, a wall surface (septum 219) in the opposite side of the septum 218, in other words, in the side opposing to the septum 218 is also formed so as to be a thick wall. Thereby, it is also prevented that the needle of the injector results in passing through the sugar fuel bag 212. In addition, it is also acceptable to provide a plate of a hard metal such as titanium on the face opposing to the septum 218. Thereby, the needle of the injector can be surely prevented from passing through the sugar fuel bag 212.

The septa 218 and 219 have the same function as that of a subcutaneous septum for the administration of commercial drug. The septum is medical equipment for drug dosage, which is subcutaneously implanted for a patient, who needs to take drug dosage into a blood vessel repeatedly, and which can mitigate a burden of inserting the needle of the injector repeatedly for the patient, by being subcutaneously implanted. When a medicine is administered, the medicine is administered through the needle which is inserted into the septa 218 and 219, and the pain and the burden to the patient can be mitigated. The septa 218 and 219 themselves are subcutaneously implanted, and accordingly are not exposed to the outside of the body. Accordingly, there is no worry of causing an infectious disease or the like.

In addition, as described above, the septa 218 and 219 are used when new sugar fuel is injected, and also has such a role as to collect old sugar fuel when the power generation has been completed and the sugar of an active material has decreased.

Here, it has been previously described that when the electric power is generated by using a noble metal as a catalyst and oxidizing sugar, the activity deterioration can be prevented from occurring due to the oxidation deterioration of the noble metal caused by the dissolved oxygen in the fuel, but the noble metal does not have activity-keeping capability for substances (protein, lipid and the like) in the living body. In other words, even the electrode of the noble metal results in immediately losing its activity as a result of adsorbing an organic matter such as the protein.

For this reason, the fuel cell 201 according to the present embodiment is structured so as to supply the sugar fuel from the outside of the living body, instead of using the body fluid and the blood of the living body. The fuel cell 201 is structured so as to have septa 218 and 219 provided in the fuel bag 212, to which the sugar fuel that does not contain impurities can be supplied from the outside of the body through a dedicated injector at low stress.

The fuel cell 201 according to the present embodiment has the fuel bag 212 provided with the above described septa 218 and 219. The fuel bag 212 having the septa 218 and 219 is subcutaneously implanted, and necessary sugar fuel can be supplied to the fuel bag from the outside of the body. The supplied sugar fuel needs to be replaced when the sugar in the fuel has been depleted. Then, the fuel in the fuel bag 212 can be replaced through the septa 218 and 219 again, and if the fuel has been replaced, the electric power can be continuously generated until the fuel is depleted again. Because the fuel is replaced through the septa 218 and 219, the burden to the patient is small and a fuel with few impurities and high purity can be replenished from the outside of the body. Accordingly, the electric power can be generated for a long time without lowering the activity of the electrode of the noble metal.

As described above, if such a noble metal electrode with a high performance can be used as to be capable of generating a high electric field thereon, such a sugar liquid for medicine as to have neutrality or weak acidity can also be used as a fuel to be supplied, and the fuel cell 201 for being implanted can be structured which uses a drip-feed solution that is a commercial medical sugar liquid, as a fuel. When the sugar liquid for medicine is used, the sugar liquid reduces sudden damage for the patient even if the bag would be disrupted, and accordingly can be safely used.

Further, the electrode is formed preferably from a noble metal, but because the fuel cell in the present embodiment can generate the electric power by supplying the sugar fuel into itself from the outside of the body, even a conventional electrode using an enzyme can prevent the activity deterioration caused by the organic matter and the like of the living body, and can be also expected to generate an electric power for a long time.

When the sugar is oxidized by using the noble metal instead of an enzyme as a catalyst, the sugar which is a fuel may be any sugar as long as the sugar has reducing properties, and an available glucide is not limited.

Specifically, the glucose is most excellent as the sugar which becomes the fuel, but a sugar having the reducing properties can be similarly used. For instance, the drip-feed solution which is sold as the medical sugar liquid contains the sugar having the reducing properties, and accordingly all the drip-feed solutions can be used.

All of the monosaccharides have the reducing properties, and accordingly all of the monosaccharides are optimal as a fuel for the fuel cell 201.

Specifically, the monosaccharides which can be used as the fuel are classified into a triose (three-carbon sugar), a tetrose (four-carbon sugar), a pentose (five-carbon sugar), a hexose (six-carbon sugar) and a heptose (seven-carbon sugar). The triose includes glyceraldehyde and dihydroxyacetone; the tetrose includes erythrose, threose and erythrulose; the pentose includes ribose, lyxose, xylose, arabinose and apiose; the hexose includes allose, talose, gulose, glucose, altrose, mannose, galactose, idose, psicose, fructose, sorbose and tagatose; and the heptose includes sedoheptulose and coriose.

Disaccharides having the reducing properties also can be used as a fuel directly in the state. The disaccharides having the reducing properties include maltose, lactose and cellobiose.

In addition, polysaccharides such as starch, glycogen and cellulose, and oligosaccharides having a molecular weight smaller than that of the polysaccharides are sugar in which monosaccharides are glucoside-bonded, and accordingly can produce monosaccharides having the reducing properties by being hydrolyzed.

For this reason, the polysaccharides and the oligosaccharide can be used as a fuel, if being converted into monosaccharides by being hydrolyzed.

Similarly, sucrose (saccharose) of the disaccharide is also a sugar in which a glucose and a fructose of monosaccharides are bonded to each other, and can be used as a fuel by being hydrolyzed, similarly to the polysaccharides and the oligosaccharides.

A metal which becomes a catalyst electrode is preferably a noble metal which has compatibility with the living body and a capability of oxidizing the sugar. However, the electrode is included in the inner part of the main body which is sealed except for having oxygen permeability, does not have a structure of being directly brought into contact with the living body, and accordingly is not required to have a high level of biocompatibility. Specifically, it is considered to form the electrode from gold, platinum or a mixture of both of the metals, or a mixture further with a co-catalytic metal such as silver and iridium.

A function of the fuel cell 201 having the above described structure will be described below.

Firstly, the fuel bag 212 is crushed in an empty state to reduce the volume so that the fuel cell can be easily implanted in the body and reduce the burden to the patient, and the fuel cell is inserted into the body. Because of this, after the fuel cell has been implanted in the body, firstly, the fuel is injected into an empty fuel bag 212. After the firstly injected fuel has been consumed, a new fuel shall be injected after the used fuel which is accumulated in the fuel bag 212 has been extracted.

<Injection operation>

The fuel is supplied into the fuel cell 201 which has been subcutaneously implanted, through septa 218 and 219 made from a silicon resin.

The operation itself at this time is the same as in HPN (Home Paremental Nutrition) with the use of an existing self-contained catheter, and the fuel liquid is injected through an inserted coreless needle (huber needle), which can suppress the perforation of the septa 218 and 219. Here, the HPN is a central venous nutrition therapy which can be conducted by the patient oneself at home.

The supplied fuel is poured into the main body of the container 211 of the fuel cell 1 from the fuel bag 212 through the injection port 213. The fuel may be injected through a syringe provided with the huber needle or also through the same equipment as the HPN. The fuel liquid which has been previously quantified is injected into the fuel bag. After the injection has been completed, the huber needle is extracted and the injection operation is finished.

<Power generation after injection>

The fuel which has been injected into the fuel cell 201 is immediately used for power generation in the fuel cell 201. Firstly, the power generation is started by using the sugar fuel which has been injected into the container 211.

Here, Fig. 6 illustrates a model view of an oxidation-reduction reaction on each electrode occurring when glucose is used as a fuel, in the fuel cell 201 which employs a metal as an oxidation catalyst for sugar. In Fig. 6, the reaction formulae in anodes (negative electrode) 225 and 226 and cathodes (positive electrode) 221 and 222 will be illustrated below. For information, in Fig. 6, the glucose is supposed to be one molecule (=2 electrons reaction) and accordingly the oxygen is supposed to be 0.5 molecules.

Here, formulae (1) and (2) are cases in which the formulae are expressed on the basis of proton transfer, and formulae (3) and (4) are cases in which the formulae are expressed on the basis of hydroxy ion transfer. It depends on the case which formulae (1) and (2) or formulae (3) and (4) are selected, and specifically on a design of the electrode, the fuel and the like that is selected according to which case the oxidation reaction system of the glucose should be handled.

$$\text{Negative electrode (oxidation): } C_6H_{12}O_6 \rightarrow C_6H_{10}O_6 + 2H^+ + 2e^-$$

$$\ldots (1)$$

$$\text{Positive electrode (reduction): } 1/2O_2 + 2H^+ + 2e^- \rightarrow$$

$$H_2O \ldots (2)$$

$$\text{Negative electrode (oxidation): } C_6H_{12}O_6 + 2OH^- \rightarrow C_6H_{10}O_6 +$$

$$2H_2O + 2e^- \ldots (3)$$

$$\text{Positive electrode (reduction): } 1/2O_2 + (H_2O) + 2e^- \rightarrow 2OH^-$$

$$\ldots (4)$$

When the formulae are described on the basis of the proton transfer, glucose ($C_6H_{12}O_6$) of the fuel is oxidized, emits an electron ($e^-$) and is converted into gluconolactone ($C_6H_{10}O_6$), on the negative electrode of the fuel cell 201. At this time, an aldehyde group having a reducing function of the glucose causes electron transfer between the aldehyde group and a hydroxyl group that has adsorbed to the electrode. Thereby, an electron is emitted and the glucose is oxidized.

On the other hand, on the positive electrode of the fuel cell 201, oxygen ($O_2$) in the air is reduced by the electron which has been produced on the negative electrode to produce water ($H_2O$). At this time, the electron ($e^-$) which has been taken out by glucose oxidation on the negative electrode cannot flow in the fuel liquid, and accordingly flows through an external circuit which electrically connects both electrodes. Thereby, the fuel cell 201 functions as a battery.

However, in order to pass the electron through the external circuit, it is necessary to migrate ions in the fuel similarly to other batteries. In the fuel cell 201, a hydrogen ion ($H^+$) migrates in the fuel liquid. When the hydrogen ion flows in the solution and the electron separately flows in the external circuit, the fuel cell forms a closed circuit, and can take out an electrical energy.

In addition, when the reaction is considered on the basis of the hydroxy ion, the reaction becomes a relationship of the formulae (3) and (4). On the negative electrode, water is produced by the proton formed by glucose oxidation and the hydroxy ion which has migrated to the electrode. On the positive electrode, the hydroxy ion is produced from oxygen, water, though water in this case is equivalent to the body fluid or the water vapor in the air, and the electron which has migrated from the negative electrode.
In other words, a difference between the reactions of the formulae (1) and (2) and the reactions of the formulae (3) and (4) is a difference between the constitution based on the migration of the proton between both electrodes and the constitution on the migration of the hydroxy ion between both electrodes, and the electrode structures are the same.

For information, Fig. 6 illustrates a reaction state of the two-electron transfer, which is a present reaction level, but glucose can provide the 24 electron reactions at maximum similarly to the phenomenon occurring in the living body, if the catalytic performance of the electrode can be further enhanced.

Here, in the case of an alkaline electrolyte in the fuel cell using the electrode with a noble metal catalyst, the hydroxyl group showing alkalinity adsorbs to the noble metal catalyst and plays a role of a reaction group for the oxidation reaction of the sugar, and passing the electron obtained by the oxidation to the electrode through the metal catalyst to which the hydroxyl group has adsorbed.
On the other hand, in the case of the neutral electrolyte in the fuel cell using the electrode with the noble metal catalyst, at least two or more noble metal catalysts are used, which enhance the electric field strength of the noble metals, lower a threshold of electron transfer in the sugar oxidation, and thereby play a role of promoting the sugar oxidation and transferring the electron to the electrode.

<Discharge operation>

When the electric power is generated and the glucose has been consumed, a huber needle having an empty syringe is inserted into the septa 218 and 219 and the used fuel is extracted, before a new fuel is injected again. After the extraction by the syringe has been completed, the syringe is replaced with a syringe having a new fuel therein, and the above described injection operation is conducted.

As described above, according to the fuel cell 201 according to the present embodiment, a fuel such as glucose is injected into the container 211 containing an electrolyte solution, through the septa 218 and 219 by a syringe or the like, and the electric power is generated in the container 211 by using this fuel. Specifically, the fuel such as the glucose, which has been injected into the container 211, emits an electron in one electrode (negative electrode) by using the noble metal such as gold, silver and platinum fixed on the surface as a catalyst, and produces a hydrogen ion (oxidation). The electron which has been emitted in the negative electrode is sent to the other electrode (positive electrode) through a wire for electrically connecting the pair of the electrodes, and the hydrogen ion migrates to the vicinity of the positive electrode in the electrolyte solution in the container 211. Thereby, on the positive electrode, the hydrogen ion which has migrated in the electrolyte solution reacts with the electron which has been sent from the negative electrode and the oxygen which has been supplied into the container 211 through the aeration portion 217 to produce water (reduction). As described above, the electric power is generated by the oxidation to be conducted in the negative electrode and the reduction to be conducted in the positive electrode, and the electric power can be supplied to equipment such as a pacemaker which is electrically connected to these electrodes.

In this case, the noble metal can function as the catalyst by being fixed on each electrode, and can generate the electric power by oxidizing the fuel such as the glucose even if the electrolyte solution is not adjusted so as to have alkalinity. Thereby, the electrolyte solution can be adjusted so as to have neutrality or weak acidity, and damage to the living body can be reduced even if the electrolyte solution would have leaked from the container 211 that has been implanted in the living body.

Here, when the electric power is generated by using sugar in the living body as a fuel, an organic matter such as protein and lipid in the living body adsorbs to the electrode even having the noble metal fixed thereon, and the noble metal results in immediately losing the activity. In contrast to this, in the case of the fuel cell 201 according to the present embodiment, a fuel with high purity can be supplied into the container 211 from the outside, and accordingly the fuel cell prevents the organic matter from adsorbing to the electrode, can reduce the deterioration of the activity of the catalyst, and can be operated for a long time.

In addition, the fuel is consumed by the generation of the electric power, but the electric power can be continuously generated by replenishing the fuel having the high purity from the outside. In other words, by enabling the fuel to be supplied from the outside through the septa 218 and 219, the container. 21 1 can reduce its size and the whole fuel cell 201 can be down-sized.

When the aeration portion 217 is formed of the carbon fluoride resin, for instance, such as ethylene tetrafluoride, the fuel cell can adequately supply oxygen to the electrode in the container 211 while preventing the leakage of the electrolyte solution from the container 211, and can efficiently conduct a reduction reaction on the electrode.

In the above described fuel cell 201, a wall of the container 211 may be formed of a carbon fluoride resin, and a portion in which the wall of the container 211 is formed so as to be locally thin may also be used as the aeration portion 217. By being structured in this way, the fuel cell can increase the amount of oxygen to be supplied to the electrode in the container 211 while preventing the leakage of the electrolyte solution from the container 211, and can enhance the reduction reaction on the electrode, in other words, the efficiency of the power generation. In addition, the fuel cell can eliminate the interface between the container 211 and the aeration portion 217, and can enhance safety when the container 211 is implanted in the living body.

The aeration portion 217 and the container 211 in Fig. 1 are described to be made of the materials different from each other, but the whole main body of the container 211 may also be constituted by the carbon fluoride resin. If the whole main body of the container 211 is constituted by the carbon fluoride resin, the container has high biocompatibility and also the interface between the different materials is eliminated, which is preferable.


[Second embodiment]

Next, the fuel cell 202 according to a second embodiment of the present invention will be described with reference to the drawings. In the description of the present embodiment, the description concerning common points to the fuel cell 201 according to the first embodiment will be omitted, and a point different from that in the first embodiment will be mainly described below. The point that the fuel cell 202 according to the present embodiment is different from the fuel cell 201 according to the first embodiment is a point that the fuel cell has a partition wall for dividing the inner part of the fuel bag into two regions provided therein.

As illustrated in Fig. 7, the fuel cell 202 according to the present embodiment is a fuel cell for being implanted in the living body, and includes a container 251 that contains an electrolyte solution therein, and a fuel bag 252 (storage portion) having a sugar fuel such as glucose stored therein. The container 251 and the fuel bag 252 are mutually connected to each other through an injection port 253 and a discharge port 254.

A septum 258 through which a fuel can be supplied and discharged from/to the outside is provided on an outer surface of the fuel bag 252. A needle protection plate 259 which is constituted by a hard metal such as titanium and prevents the insertion of an injector is provided on the face which opposes the septum 258.

As illustrated in Fig. 8, a partition wall 250 which divides the fuel bag 252 into two regions (body surface tank 265 and inner body tank 266) is provided in the inner part of the fuel bag 252.

The partition wall 250 is constituted by a heat insulating material, divides the inner part of the fuel bag 252 into one face side in which the septum 258 is provided and the other face side which opposes the one face, and opens in an upper end edge of the fuel bag 252. The injection port 253 and the discharge port 254 are provided in the body surface tank 265 and the inner body tank 266 which are divided by the partition wall 250, respectively, and the injection port 253 is provided in a lower part of the fuel bag 252 and the discharge port 254 is provided in an upper part of the fuel bag 252, respectively.

Fins 261 and 262 are provided on the outer surface of the fuel bag 252, and function as a heat exchanger which exchanges heat between the tissue in the living body and a sugar fuel in the inner part of the fuel bag 252. These fins 261 and 262 are provided in the body surface side and the inner body side, respectively.

The function of the fuel cell 202 having the above described structure will be described below.

The fuel cell 202 is used in a form of being subcutaneously implanted in the living body. At this time, the fuel cell 202 is implanted so that the one face side in which the septum 258 is provided becomes the body surface side and the other face side which opposes the septum 258 becomes the inner body side. In the body surface side in which the septum 258 is provided, the fuel bag 252 and the fin 261 in the body surface side are kept at a temperature in the vicinity of the body surface. On the other hand, in the other face side which opposes the septum 258, the fuel bag 252 and the fin 262 in the inner body side are kept at a temperature of the inner body.

Here, there is a temperature difference among the sites in the living body of a person. In general, the temperature of the body surface is approximately 35 to 35.5°C, and the temperature of the inner body is 37 to 37.5°C. In the body center part in particular, the body temperature is known to be approximately 40°C. The fuel cell 202 according to the present embodiment circulates (convects) the sugar fuel by using this temperature difference as energy.

The sugar fuel which has been supplied via the septum 258 is dividedly supplied to the body surface tank 265 and the inner body tank 266 each of the fuel bag 252. The sugar fuel which has flowed into the inner body tank 266 is warmed in the high-temperature body, and forms a warm flow 263. On the other hand, the sugar fuel which has flowed into the body surface tank 265 is cooled by the body surface, and forms a cool flow 264.

The sugar fuel which has been warmed in the fin 262 and the inner body tank 266 in the inner body side forms the warm flow 263, moves up through the inner body tank 266, passes through an aperture of an end edge of the partition wall 250, and flows into the body surface tank 265. On the other hand, the sugar fuel which has been cooled in the fin 261 and the body surface tank 265 in the body surface side forms the cool flow 264, moves down through the body surface tank 265, and flows out to the container 251 from the injection port 253 provided in a lower part of the body surface tank 265. Thereby, in the fuel bag 252, the natural convection of the sugar fuel occurs due to the body temperature difference in the living body, the sugar fuel can be stably supplied and discharged to/from the container 251.

Specifically, the specific gravity of the sugar fuel changes due to the temperature difference between the body surface tank 265 and the inner body tank 266, and the convection of the sugar fuel occurs originating in such an action as to keep the temperature in equilibrium by the difference between the specific gravities of the sugar fuel. In the fuel cell 2, it is important for electric power generation over a long period of time to migrate the sugar fuel of an energy source to the electrode and migrate the product produced after the completion of the electric power generation away from the electrode. This principle is similar, for instance, to the principle that it is important for stably operating an internal combustion engine for a long time to supply gasoline which is a fuel and also efficiently exhaust a combustion gas. At this time, ion migration originating in a supporting electrolyte in the fuel actually occurs according to the electric current obtained by the oxidation of the sugar on the interface of the electrode. However, the ion migration which is the migration of a substance is slower than the electron transfer and has a large resistance. The diffusion movement of the ion migration becomes the largest internal resistance of the fuel cell 2.

For this reason, the fuel cell 202 according to the present embodiment supplies and discharges the fuel for the operation for a long time, gives the convection due to the temperature of the living body to the whole fuel, thereby can reduce the resistance originating in the ion migration and can provide an operation system having a high power-generating capability and a long life.

Here, because the sugar is not an electrolyte, the convection and the diffusion of the sugar itself cannot be expected. The sugar fuel diffuses due to the concentration change which is caused by the electric power generation, but the sugar having an extremely large molecular weight becomes a large resistance in the diffusion process. The large diffusion resistance makes it difficult for the fuel cell to stably provide a large electric current, and accordingly by making the fuel to be diffused by using the body temperature as in the present embodiment, the fuel can be more efficiently consumed without producing waste, and the electric power can be generated for a long time even by the fuel having the same concentration.

As described above, in the fuel cell 202 according to the present embodiment, the partition wall 250 divides the inner part of the fuel bag 252 into one face side in which the septum 258 is provided and the other face side which opposes the one face, and thereby can keep the temperature of the sugar fuel in the other face side (inner body side) higher than that of the sugar fuel in the one face side (body surface side) in which the septum 258 is provided, when being implanted in the body. Thus, the fuel cell forms the temperature difference between the sugar fuel in the inner body side and the sugar fuel in the body surface side, thereby promotes the convection of the sugar fuel in the fuel bag 252, and can supply the sugar fuel to the container 251 from the fuel bag 252 through the injection port 253 by using this convection.

Further, the fuel cell has the fins 261 and 262 which exchange the heat between the outside and the inside of the fuel bag 252 provided on the outer surface of the fuel bag 252, thereby can efficiently form the temperature difference between the sugar fuel in the inner body side and the sugar fuel in the body surface side, promotes the convection of the sugar fuel in the fuel bag 252 and can efficiently supply the sugar fuel to the container 251 from the fuel bag 252.

[Modified example]

As for a modified example of the fuel cell 202 according to each of the above described embodiments, as illustrated in Fig. 9 to Fig. 11, the fuel bag 252 and the container 251 are separated from each other, and the fuel bag 252 may also be connected to the container 251 by pipes (flow channel) 271 and 272. The pipes 271 and 272 adopt, for instance, a Teflon (registered trademark) hose having flexibility and biocompatibility.

The fuel cell 202 according to the present modified example has the fuel bag 252 and the container 251 which are formed so as to be a separated type, accordingly can enhance the flexibility of the layout when the fuel bag 252 and the container 251 are implanted in the living body, and can implant each of the fuel bag 252 and the container 251 in an empty space of the living body.

The embodiment of the present invention was described in detail above with reference to the drawings, but the specific structure is not limited to this embodiment, and the change in a design is also included in such a range as not to deviate from the scope of the present invention.

In addition, in the embodiment of the present invention, a hydrogen ion permeable membrane is used, but the ion permeable membrane is not limited to the hydrogen ion permeable membrane and may be any ion permeable membrane. For instance, an anion permeable membrane which makes a hydroxy ion (anion) permeate therethrough may also be used instead of the membrane which makes the hydrogen ion (proton) permeate therethrough.

For instance, in each of the embodiments, the fuel cell has been described assuming that the fuel cell has the container and the fuel bag provided therein, but the fuel cell may also have a septum provided on the container itself instead of having the fuel bag.

Further, in the second embodiment, the partition wall 250 may be provided so as to open in a lower end edge of the fuel bag 252, at the same time, the injection port 253 may also be provided in an upper part of the inner body tank 266, and the discharge port 254 may also be provided in a lower part of the body surface tank 265.

Furthermore, in the above described modified example, the modified example was described on the basis of the fuel cell 202 according to the second embodiment, but the container and the fuel bag may be formed so as to be a separated type in the fuel cell 201 according to the first embodiment.

[Third embodiment]

A battery 1 according to a third embodiment of the present invention will be described below with reference to the drawings.

The battery 1 according to the present embodiment is, for instance, an enzyme type glucose fuel cell, as illustrated in Fig. 12, and includes: a container 10 which contains an electrolyte solution containing glucose therein; a partition wall 13 which divides the container 10 and forms a cell 11 and a cell 12; a positive electrode 14 and a negative electrode 15 arranged in each of the cells 11 and 12, respectively; an injection port 16 provided on an outer surface of the container 10; a continuous hole 18 provided in the partition wall 13; and a slit valve (flow-channel opening/closing portion) 17 provided in the continuous hole 18.

The container 10 is a watertight container, and is structured so as to contain an electrolyte solution which is injected from the outside through the injection port 16, a sugar fuel such as glucose and an enzyme such as nicotinamide adenine dinucleotide (NADH) as a mediator therein.

The partition wall 13 is formed in the inner part of the container 10 so as to be approximately parallel to the upper face and the lower face of the container 10, and is structured so as to divide the inner part of the container 10 and form the cell 11 and the cell 12 which have an approximately equal volume.

The positive electrode 14 and the negative electrode 15 are provided as a pair in the inner part of each of the cell 11 and the cell 12.

The negative electrode 15 is a carbon electrode having an enzyme, for instance, such as glucose dehydrogenase immobilized thereon. The negative electrode 15 is formed so as to oxidize the glucose in the electrolyte solution on its surface while using the enzyme immobilized on the surface of the electrode as a catalyst, emits an electron to the negative electrode 15, and also produces a hydrogen ion.

The positive electrode 14 is a carbon electrode having an enzyme, for instance, such as bilirubin oxidase immobilized thereon. The positive electrode 14 is formed so as to make the hydrogen ion which has migrated in the electrolyte solution, the electron which has been sent from the negative electrode 15 and oxygen which exists in the electrolyte solution or has been supplied from the outside react with each other on the surface of the positive electrode while using the enzyme immobilized on the surface of the electrode as a catalyst, and to produce water (reduction).

The container 10 has an aperture 20 provided therein, and the aperture 20 has an air permeable waterproof sheet 19 having air permeability and waterproofness provided therein. The air permeable waterproof sheet 19 is formed from a carbon fluoride resin, for instance, such as polytetrafluoroethylene (ethylene tetrafluoride).
The positive electrode 14 is arranged so as to abut on or be close to the air permeable waterproof sheet 19. By being structured in this way, the container 10 can supply oxygen from the outside of the container 10 to the positive electrode 14 while preventing the electrolyte solution and the like from leaking from the container 10.

The positive electrode 14 arranged in the cell 11 and the negative electrode 15 arranged in the cell 12 are connected by a conducting wire 23. A positive electrode terminal 21 for being connected to the external electronic equipment is connected to the positive electrode 14 arranged in the cell 12. A negative electrode terminal 22 for being connected to the external electronic equipment is connected to the negative electrode 15 arranged in the cell 11. By being structured in this way, the cell 11 and the cell 12 are structured to be serially connected and supply the electric power to the electronic equipment which has been connected to the positive electrode terminal 21 and the negative electrode terminal 22.

The injection port 16 is constituted by an elastic member, for instance, such as silicon, and has a slit 25 for penetrating a pipe therethrough such as a syringe needle formed therein, as illustrated in Fig. 13. The injection port 16 is provided approximately in the center in the upper face of the container 10, when the container 10 is viewed as the plane. By being structured in this way, the injection port 16 is structured so that the electrolyte solution can be injected into the container 10 from the outside through the pipe such as the syringe needle when the pipe has been inserted into the slit 25, as illustrated in Fig. 14.

The continuous hole 18 is a hole provided approximately in the center of the partition wall 13, when the partition wall 13 is viewed as the plane, and is structured so as to make the cell 11 and the cell 12 communicate with each other. The slit valve 17 is a valve which is constituted by an elastic member, for instance, such as silicon, similarly to the injection port 16, and has the slit 25 for making the pipe such as the syringe needle penetrate therethrough formed therein. The slit valve 17 is provided approximately in the center of the partition wall 13, when the partition wall 13 is viewed as the plane, as illustrated in Fig. 13. In other words, the slit valve 17 is arranged on the straight line which passes through the injection port 16 and the continuous hole 18.

The slit 25 of the injection port 16 and the slit valve 17 is structured to open in a lemon shape, on the condition that the syringe needle 27 has been inserted into the slit 25, as illustrated in Fig. 15. By being structured in this way, as illustrated in Fig. 16, a gap is formed between the slit 25 and the syringe needle 27, and the air and/or the electrolyte solution can be preferably passed from the gap, when the syringe needle 27 has been inserted into the slit 25.

As for the slit 25 of the injection port 16 and the slit valve 17, members in both sides of the slit 25 incline toward the direction toward which the syringe needle 27 is inserted (downward direction of the page in Fig. 12) and come in contact with each other. When the slit 25 is formed into such a shape, the members in both sides of the slit 25 more strongly come in close contact with each other by a pressure which the members receive from the electrolyte solution filled in the cell 11 and the cell 12, when the syringe needle 27 has been extracted from the slit 25, and the water-tightness of the injection port 16 and the slit valve 17 can be enhanced.

The electrolyte solution to be supplied to the container 10 is injected by a syringe 9, as illustrated in Fig. 14. The syringe 9 includes a cylindrical member 29 having an opened bottom surface, a movable piston portion 28 to be inserted into an inner part of the cylindrical member 29, and a syringe needle (pipe) 27 which is connected to an upper face (face which opposes the bottom surface) of the cylindrical member 29. The syringe needle 27 communicates with the inner part of the cylindrical member 29, and has an opened tip. The syringe 9 is structured so as to discharge a fluid contained in the inner part of the cylindrical member 29 from the tip of the syringe needle 27 when the piston portion 28 of the syringe 9 is pressed.

By having such a structure, as illustrated in Fig. 14, the syringe 9 can inject the electrolyte solution into the container 10 (cell 12) through the syringe needle 27 in a state where the electrolyte solution is contained in the cylindrical member 29 of the syringe 9, by making the syringe needle 27 penetrate through the injection port 16 and the slit valve 17. In addition, the syringe makes the syringe needle 27 expand the slit 25 of the slit valve 17 and open the flow channel between the cell 11 and the cell 12, and can supply the electrolyte solution from the cell 12 into the cell 11.

The syringe is also structured so that the slit 25 is blocked by an elastic force of the slit valve 17 when this syringe needle 27 is extracted from the injection port 16 and the slit valve 17, and that the flow channel between the cell 11 and the cell 12 is closed. In other words, the slit valve 17 is structured so as to open the flow channel between the cell 11 and the cell 12 when the electrolyte solution is injected into the container 10, and close the flow channel between the cell 11 and the cell 12 after the electrolyte solution has been injected into the container 10.

A function of the battery 1 having the above described structure will be described below. As illustrated in Fig. 14, when the syringe needle (pipe) 27 of the syringe 9 is inserted into the injection port 16 and the slit valve 17, the syringe needle 27 expands the slit 25 of the injection port 16 and the slit valve 17 which has been formed of the elastic body and penetrates from the injection port 16 through the cell 11, and the tip of the syringe needle 27 is inserted into the cell 12, as illustrated in Fig. 15.

In this state, when the piston portion 28 of the syringe 9 is pressed and the pressure is applied to the electrolyte solution (including the sugar fuel such as glucose) contained in an inner part of the syringe 9, as illustrated in Fig. 16, the electrolyte solution in the inner part of the syringe 9 passes through the syringe needle 27, and is supplied into the cell 12 from the tip of the syringe needle 27.

The electrolyte solution which has been supplied into the cell 12 from the syringe 9 fills the inner part of the cell 12. At this time, air in the inner part of the cell 12 passes through the slit 25 of the slit valve 17, and is discharged to the cell 11, as illustrated in Fig. 16. The air in the inner part of the cell 11 passes through the slit 25 of the injection port 16, and is discharged to the outside of the container 10 (cell 11).

When the electrolyte solution which has been supplied from the syringe 9 fills the inner part of the cell 12, the electrolyte solution in the cell 12 is supplied to the cell 11 from a gap formed in the expanded slit 25 of the slit valve 17, as illustrated in Fig. 16. Thus, the electrolyte solution is injected into the cell 11 and the cell 12.

When the operation of injecting the electrolyte solution into the cell 11 and the cell 12 has been finished, the syringe needle 27 is extracted from the injection port 16 and the slit valve 17. At this time, as illustrated in Fig. 17, the slit 25 is blocked by the elastic force of the slit valve 17, and the flow channel between the cell 11 and the cell 12 is closed. In addition, the slit 25 is blocked by the elastic force of the injection port 16, and the electrolyte solution is prevented from leaking to the outside from the container 10 (cell 11).

In this state, the electric power is generated in the cell 11 and the cell 12. Specifically, the glucose in the electrolyte solution in the cell 11 and the cell 12 emits an electron on the negative electrode 15 by using glucose dehydrogenase as a catalyst, which has been immobilized on the surface of the electrode, and also produces a hydrogen ion (oxidation). The electron which has been emitted to the negative electrode 15 is sent to the positive electrode 14 through a wire for electrically connecting the negative electrode 15 with the positive electrode 14.

The produced hydrogen ion migrates to the vicinity of the positive electrode 14 in the electrolyte solution in the cell 11 and the cell 12. Thereby, the hydrogen ion which has come to the positive electrode 14 while migrating in the electrolyte solution, the electron which has been sent from the negative electrode 15 and the oxygen which has permeated the air permeable waterproof sheet 19 from the outside of the container 10 react with each other on the positive electrode 14 to produce water (reduction). As described above, an electric power is generated by the oxidization to be conducted in the negative electrode 15 and the reduction to be conducted in the positive electrode 14, and the electric power is supplied to electronic equipment which is electrically connected to these electrodes.

As described above, according to the battery 1 according to the present embodiment, when the electrolyte solution is injected into the container 10, and when the syringe needle 27 is inserted into the container 10 through the injection port 16, the flow channel between the cell 11 and the cell 12 is opened by the slit valve 17, and the cell 11 and the cell 12 communicate with each other. In this state, the electrolyte solution is injected into the cell 11 and the cell 12 from the syringe 9. After the electrolyte solution has been injected into the container 10, the syringe needle 27 is extracted from the injection port 16 and the slit valve 17, and then the flow channel between the cell 11 and the cell 12 is closed by the slit valve 17. Thereby, the fuel cell is formed so as to have the cell 11 and the cell 12 independent from each other.

In other words, the battery 1 according to the present embodiment can easily inject the electrolyte solution into the cell 11 and the cell 12 in one injection operation, and also can prevent the electric charge from migrating between the cell 11 and the cell 12 after the electrolyte solution has been injected. Thereby, the battery prevents the voltage from being lowered due to the migration of the electric charge between the cells when the cell 11 and the cell 12 are serially connected to each other, and can provide a desired voltage.

In the present embodiment, the structure may also be employed in which the fuel bag or the periphery of the electrode is integrated, as in the first or the second embodiment, for instance.

[Fourth embodiment]

Next, a battery 2 according to a fourth embodiment of the present invention will be described below with reference to the drawings. In the description of the present embodiment, the description concerning common points to the third embodiment will be omitted, and a different point will be mainly described below.

The point at which the battery 2 according to the present embodiment is different from the battery 1 according to the third embodiment is the shape of the slit of the injection port 16 and the slit valve 17.

The battery 2 according to the present embodiment has the same structure as that of the battery 1 according the first embodiment, except the shape of the slit of the injection port 16 and the slit valve 17, as illustrated in Fig. 18. The injection port 16 and the slit valve 17 have a pinhole 29 which makes the syringe needle 27 penetrate therethrough, in place of the slit 25, as illustrated in Fig. 19.

When a silicon rubber material which is sensitive to a tear, for instance, is adopted for the injection port 16 and the slit valve 17, the tear in the slit grows, and as a result, the growth occasionally results in lowering the water-tightness of the injection port 16 and the slit valve 17.

In such a case, as in the battery 2 according to the present embodiment, it is effective to provide the pinhole 29 instead of the slit in the injection port 16 and the slit valve 17.

In this case, the syringe needle 27 is desirably formed so as to have a shape in which an outer peripheral face is recessed toward the inside (inward direction in a radial direction), as illustrated in Fig. 20. In other words, a transverse sectional shape of the syringe needle 27 is desirably formed into such a transverse sectional shape in which a recess 32 is provided in an envelope curve 31 that envelops the outer shape, for instance, such as a star shape, instead of a circular shape as illustrated in Fig. 16. Thus, when the transverse sectional shape of the syringe needle 27 is structured in this way, the cell 11 and the cell 12 can communicate with each other through the recess 32, when the syringe needle 27 has been inserted into the container 10.

The battery 2 having the above described structure according to the present embodiment can easily inject the electrolyte solution into the cell 11 and the cell 12 in one injection operation, while preventing the water-tightness from being lowered by the enlargement of the tear of the slit of the injection port 16 and the slit valve 17. In addition, after the syringe needle 27 has been extracted from the injection port 16 and the slit valve 17, the battery can prevent the electric charge from migrating between the cell 11 and the cell 12. Thereby, the battery prevents the voltage from being lowered due to the migration of the electric charge between the cells when the cell 11 and the cell 12 are serially connected to each other, and can provide a desired voltage.

It is also acceptable as illustrated in Fig. 21 to block the tip of the syringe needle 27 and also provide apertures 35 and 36 at such respective positions as to correspond to the cell 11 and the cell 12, when the syringe needle 27 is inserted into the container 10.

By being structured in this way, the syringe needle enables an operation of injecting the electrolyte solution into the cell 11 and the cell 12 to be conducted at the same time, which can shorten a necessary time for the injection operation.

In the present embodiment, the structure may also be employed in which the fuel bag or the periphery of the electrode is integrated, as in the first or the second embodiment, for instance.

[Fifth embodiment]

Next, a battery 3 according to a fifth embodiment of the present invention will be described below with reference to the drawings. In the description of the present embodiment, the description concerning common points to each of the above described embodiments will be omitted, and a different point will be mainly described below.

The point at which the battery 3 according to the present embodiment is different from the batteries 1 and 2 according to each of the above described embodiments is the quantity and the arrangement of partition walls which divide each cell.

The battery 3 according to the present embodiment includes partition walls 44 and 45 for dividing the inner part of the container 10 to form three cells 41, 42 and 43 which have an approximately equal volume, as illustrated in Fig. 22. The partition wall 44 has a bottom wall 44a formed so as to be approximately parallel to the upper face and the lower face of the container 10, and a side wall 44b formed so as to be approximately parallel to a side face of the container 10. The partition wall 45 has a bottom wall 45a formed so as to be approximately parallel to the upper face and the lower face of the container 10, and a side wall 45b formed so as to be approximately parallel to the side face of the container 10.

The bottom wall 45a is arranged in a lower direction of the bottom wall 44a, and the side wall 45b is arranged in a more outward part in the radial direction than the side wall 44b. In other words, the partition wall 45 divides the inner part of the container 10 into the cell 43 and the other portion (cell 41 and cell 42), and the partition wall 44 divides the other portion into the cell 41 and the cell 42.

The positive electrode 14 and the negative electrode 15 are provided as a pair in the inner part of each of the cells 41, 42 and 43.

The positive electrode 14 arranged in the cell 41 and the negative electrode 15 arranged in the cell 42 are connected by a conducting wire 23. The positive electrode 14 arranged in the cell 42 and the negative electrode 15 arranged in the cell 43 are connected by a conducting wire 23. A positive electrode terminal 21 for being connected to exterior electronic equipment is connected to the positive electrode 14 arranged in the cell 43. A negative electrode terminal 22 for being connected to the exterior electronic equipment is connected to the negative electrode 15 arranged in the cell 41. By being structured in this way, the three cells 41, 42 and 43 are structured so as to be serially connected, and supply the electric power to the electronic equipment which has been connected to the positive electrode terminal 21 and the negative electrode terminal 22.

The injection port 16 is provided on an outer peripheral face of the container 10. Slit valves 17 are provided in the side walls 44b and 45b, respectively. These injection port 16 and slit valves 17 are arranged on the straight line. In other words, the injection port 16 and slit valves 17 are structured so that the syringe needle 27 penetrates the injection port 16 and the slit valves 17 when the syringe needle 27 of the syringe 9 is inserted toward the inside from the outer peripheral face of the container 10.

The battery 3 having the above described structure according to the present embodiment can shorten a distance between the outer peripheral face of the container 10 and the side wall 45b and a distance between the side wall 45b and the side wall 44b, and can shorten the length of the syringe needle 27 of the syringe 9 necessary for injecting the electrolyte solution into the cells 41, 42 and 43.

The battery 3 according to the present embodiment may include a bottom wall 44c which is connected to the side wall 44b and is formed so as to be approximately parallel to the upper face and the lower face of the container 10, and a bottom wall 45c which is connected to the side wall 45b and is formed so as to be approximately parallel to the upper face and the lower face of the container 10, in a lower part of the bottom wall 44c, as illustrated in Fig. 23.

In this case, when the slit valves 17 are provided on the bottom walls 44c and 45c, respectively, and these slit valves 17 and injection port 16 are arranged on the straight line, the length of the syringe needle 27 of the syringe 9 necessary for injecting the electrolyte solution into the cells 41, 42 and 43 can be thereby shortened, similarly to the battery 3 according to the present embodiment.

In addition, in the present embodiment, the structure may also be employed in which the fuel bag or the periphery of the electrode is integrated, as in the first or the second embodiment, for instance.

[Sixth embodiment]

Next, a battery 4 according to a sixth embodiment of the present invention will be described below with reference to the drawings. In the description of the present embodiment, the description concerning common points to each of the above described embodiments will be omitted, and a different point will be mainly described below.

The battery 4 according to the present embodiment is, for instance, a car battery which is constituted by six cells, as illustrated in Fig. 24.

As illustrated in Fig. 24 and Fig. 25, the battery 4 has an injection port 51 for injecting the electrolyte solution therethrough into each cell provided on each cell, and has a manual valve (flow-channel opening/closing portion) 53 which opens/closes the flow channel between each cell, and a manual cock 52 which is connected to the manual valve 53 and operates the manual valve 53 provided between each cell.

By having the above described structure, the battery 4 can block the flow channel between the cells by operating the manual cock 52 and closing the manual valve 53, and can make the adjacent cells communicate with each other by operating the manual cock 52 and opening the manual valve 53.

The positive electrode 14 and the negative electrode 15 are provided as a pair in the inner part of each cell. These electrodes are serially connected to each other, and constitute a battery in which the six cells are serially connected to each other.

A function of the battery 4 having the above described structure will be described below.

Firstly, as illustrated in Fig. 27, the manual cock 52 is operated, and all of the manual valves 53 are opened which are provided in each of five partition walls.

Next, a lid (not shown) of any one of injection ports 51 out of the six injection ports 51 provided in each cell is opened, and the electrolyte solution is injected into the cell. Thereby, the electrolyte solution is injected into all of the cells by one injection operation.

When the injection of the electrolyte solution has been completed, the manual cock 52 is operated, and all of the five manual valves 53 are closed, as illustrated in Fig. 26. Thereby, all the cells are blocked, and the battery is constituted in which all the cells are serially connected to each other.

As described above, according to the battery 4 according to the present embodiment, each cell can communicate with each other by the operation of opening the manual valve 53 when the electrolyte solution is injected into the container, and the communication between each cell can be prohibited by the operation of closing the manual valve 53, after the electrolyte solution has been injected into the container. Thereby, the electrolyte solution can be easily injected into the plurality of the cells by one injection operation, and also the electric charge can be prevented from migrating between the cells after the electrolyte solution has been injected into the container.

In the present embodiment, the structure can also be employed in which the fuel bag or the periphery of the electrode is integrated, as in the first or the second embodiment, for instance.

[Seventh embodiment]

Next, a battery 5 according to a seventh embodiment of the present invention will be described with reference to the drawings. In the description of the present embodiment, the description concerning common points to each of the above described embodiments will be omitted, and a different point will be mainly described below.

The battery 5 according to the present embodiment is a water battery which functions as a battery when water, for instance, is injected thereinto.

The battery 5 according to the present embodiment has a cylindrical shape, and has an injection port 62 for injecting water therethrough provided approximately in the central part of the upper face, as illustrated in Fig. 28. In addition, a plurality of air permeable waterproof sheets 19 are arranged on an outer peripheral face of the battery 5.

In the battery 5 according to the present embodiment, partition walls 65 and 66 divide the container 10 having the cylindrical shape and form a plurality of cells, as illustrated in Fig. 29. Specifically, the partition wall 65 having the cylindrical shape is arranged approximately in the center of the container 10, and forms a common flow channel 60 approximately in the center of the container 10. The six partition walls 66 are arranged so as to radially extend to the outward part in the radial direction from the partition wall 65, and form six cells 61 having an approximately equal volume. These cells 61 are formed so as to be adjacent in the outside of the common flow channel 60.

A continuous hole which makes the common flow channel 60 and the cell 61 communicate with each other is provided each in a space between the common flow channel 60 and the cell 61, in other words, in the partition wall 65. Each continuous hole is provided with a non-return valve 67 which passes the fluid from the common flow channel 60 into the cell 61, and on the other hand, prohibits the fluid from flowing into the common flow channel 60 from the cell 61. The non-return valve 67 is constituted, for instance, by a silicon rubber, and is formed into a duck bill shape, as illustrated in Fig. 30. For information, the non-return valve 67 may also have the same structure as that of the valve rubber of a bicycle.

The positive electrode 14 and the negative electrode 15 are provided as a pair each in the inner part of the cell 61. These cells 61 constitute a battery in which the positive electrode 14 is connected with the negative electrode 15 of an adjacent cell 61 and the six cells 61 are serially connected.

The negative electrode 15 is a magnesium electrode, and the positive electrode 14 is a carbon electrode. The insulating material containing a salt content is sandwiched between the positive electrode 14 and the negative electrode 15. By having such a structure, the battery 5 constitutes a water battery which does not discharge electricity so long as water is not injected therein and can be preserved for a long period.

The injection port 62 is constituted, for instance, by a silicon rubber, and has a pinhole 63 at a vertex of the conical shape.

The injection port 62 may have a structure of having a septum therein which is made from a silicon rubber having no pinhole, instead of the structure having the pinhole 63, in which a syringe needle 27 pierces the septum and penetrates through the septum.

An aperture is provided on the outer peripheral face of the container 10 in each cell, and the air permeable waterproof sheet 19 is provided on each aperture.

A function of the battery 5 having the above described structure will be described below.

As illustrated in Fig. 31, the syringe needle 27 is inserted into the pinhole 63 of the injection port 62 in a state in which a syringe 9 is filled with water.

Next, the water in the syringe 9 is injected into the common flow channel 60 from the syringe needle 27.

When the common flow channel 60 is filled with the water, each non-return valve 67 is opened by a water pressure in the common flow channel 60, and the water simultaneously flows into each cell.

At this time, the gas which has originally filled the cell 61 is exhausted to the outside through the air permeable waterproof sheet 19 provided in each cell 61.

After the injection operation to all of the cells 61 has been completed, the syringe needle 27 is extracted. Thereby, each non-return valve 67 is closed, and the flow channels between the common flow channel 60 and each cell 61 are closed. Thereby, the whole battery 5 is structured as a battery in which all of the cells 61 are formed so as to be an independent battery and all of the cells 61 are serially connected.

As described above, according to the battery 5 according to the present embodiment, the flow channels between the common flow channel 60 and each cell 61 are opened by the non-return valve 67, when the water is supplied to the common flow channel 60 through the injection port 62. After the operation of injecting the water into the common flow channel 60, the flow channels between the common flow channel 60 and each cell 61 are closed by the non-return valve 67. Thereby, the water can be easily injected into the plurality of the cells 61 through the common flow channel 60 by one injection operation, and the electric charge can also be prevented from migrating between the common flow channel 60 and each cell 61 and between the cells 61.

By providing the non-return valves 67 between the common flow channel 60 and each cell 61, the battery can pass the water to the cell 61 from the common flow channel 60 without needing the opening/closing operation of the valve, and can also prohibit the water from flowing to the common flow channel 60 from the cell 61.

By arranging each cell 61 so as to be adjacent in the outside of the common flow channel 60, the battery can arrange many cells 61 so as to be adjacent to one common flow channel 60, and can be down-sized.

The battery 5 according to the present embodiment may have an aperture provided on an upper face of each cell 61, and may also have the air permeable waterproof sheet 19 arranged on this aperture, as illustrated in Fig. 32.

By being structured in this way, the gas can be adequately exhausted from each cell 61 when the water is injected into each cell 61, and the water-injection operation to each cell 61 can be facilitated, as illustrated in Fig. 33.

In the structure according to the present embodiment, the structure may also be employed in which the fuel bag or the periphery of the electrode is integrated, as in the first or the second embodiment, for instance.

[Eighth embodiment]

Next, a battery 6 according to an eighth embodiment of the present invention will be described below with reference to the drawings. In the description of the present embodiment, the description concerning common points to each of the above described embodiments will be omitted, and a different point will be mainly described below.

The battery 6 according to the present embodiment is, for instance, an alkali type glucose-fuel cell.

In the battery 6 according to the present embodiment, as illustrated in Fig. 35, the inner part of the container 10 is divided by a partition wall 72 which is provided so as to be approximately parallel to the side face of the container 10 and a partition wall 74 which is provide so as to be approximately parallel to the bottom face of the container 10 to have four chambers formed of a first oxidation electrode chamber 81 and a second oxidation electrode chamber 82 in which an oxidation reaction is conducted, and a first reduction electrode chamber 83 and a second reduction electrode chamber 84 in which a reduction reaction is conducted.

An injection port 79 which is constituted by a silicon rubber, for instance, and has a pinhole on the vertex of the conical shape, is provided on an upper face of the first oxidation electrode chamber 81.

A non-return valve 73 which permits a fluid to flow only in the direction from the first oxidation electrode chamber 81 into the second oxidation electrode chamber 82 is arranged in the partition wall 74 which divides the first oxidation electrode chamber 81 and the second oxidation electrode chamber 82.

An injection port 78 which is constituted by a silicon rubber, for instance, and has the pinhole on the vertex of the conical shape, is provided on an upper face of the first reduction electrode chamber 83.

A non-return valve 73 which permits a fluid to flow only in the direction from the first reduction electrode chamber 83 into the second reduction electrode chamber 84 is arranged in the partition wall 74 which divides the first reduction electrode chamber 83 and the second reduction electrode chamber 84.

Each non-return valve 73 is arranged at a position which is deviated from the insertion direction of the syringe needle 27 so that the non-return valve is not pierced by the syringe needle 27 when the syringe needle 27 has been inserted into each of the injection ports 78 and 79.

An oxidation electrode (negative electrode) 85 which is constituted by a carbon paper having gold fine particles fixed thereon is arranged in each of the first oxidation electrode chamber 81 and the second oxidation electrode chamber 82.

An aperture is provided in the first oxidation electrode chamber 81 and the second oxidation electrode chamber 82, and an air permeable waterproof sheet 19 is provided on this aperture.

A reduction electrode (positive electrode) 86 which is constituted by a stainless net electrode having manganese oxide fixed thereon is arranged in each of the first reduction electrode chamber 83 and the second reduction electrode chamber 84. Each of the first reduction electrode chamber 83 and the second reduction electrode chamber 84 has an aperture provided in the vicinity of the outside of the reduction electrode 86, and the air permeable waterproof sheet 19 is provided in this aperture. A proton permeable type of a solid electrolyte membrane 75 is provided in the vicinity of the inner side of the reduction electrode 86.

A negative electrode terminal 22 is connected to the oxidation electrode 85 of the first oxidation electrode chamber 81, and a positive electrode terminal 21 is connected to the reduction electrode 86 of the second reduction electrode chamber 84. The oxidation electrode 85 of the second oxidation electrode chamber 82 and the reduction electrode 86 of the first reduction electrode chamber 83 are connected by a conducting wire 23. By being structured in this way, the battery is structured as a whole so that the two cells are serially connected and supply the electric power to the electronic equipment which has been connected to the positive electrode terminal 21 and the negative electrode terminal 22.

The battery 6 has a manual valve 71 which opens/closes the flow channel between the first oxidation electrode chamber 81 and the first reduction electrode chamber 83, provided in the partition wall 72 which divides the first oxidation electrode chamber 81 and the first reduction electrode chamber 83.

The battery 6 has a manual valve 71 which opens/closes the flow channel between the second oxidation electrode chamber 82 and the second reduction electrode chamber 84, provided in the partition wall 72 which divides the second oxidation electrode chamber 82 and the second reduction electrode chamber 84.

These manual valves 71 are connected by a link (connection mechanism) 77, and are structured so that when one manual valve 71 is operated, the other manual valve 71 works in synchronization with the operation, as illustrated in Fig. 34.

A drain cock 76 for discharging the fuel liquid and the like which have been supplied to the inner part is provided in a bottom face of the second oxidation electrode chamber 82. The drain cock 76 is used in a state of being closed, except the time when the fuel liquid is discharged.

A function of the battery 6 having the above described structure will be described below.

Firstly, the manual valve 71 and the drain cock 76 are closed as a preparatory stage.

Next, as illustrated in Fig. 36, a syringe needle 27 is inserted into the injection port 79 of the first oxidation electrode chamber 81, and a glucose solution B is injected from a syringe 9. When the first oxidation electrode chamber 81 is filled with the glucose solution B, the non-return valve 73 is opened by a pressure of the glucose solution B, and the glucose solution B is injected also into the second oxidation electrode chamber 82. At this time, the gas which has originally filled the first oxidation electrode chamber 81 and the second oxidation electrode chamber 82 is discharged to the outside through the air permeable waterproof sheet 19.

Next, the syringe needle 27 is inserted into the injection port 78 of the first reduction electrode chamber 83, and an alkaline solution A is injected from the syringe 9. When the first reduction electrode chamber 83 is filled with the alkaline solution A, the non-return valve 73 is opened by a pressure of the alkaline solution A, and the alkaline solution A is injected also into the second reduction electrode chamber 84. At this time, the gas which has originally filled the first reduction electrode chamber 83 and the second reduction electrode chamber 84 is discharged to the outside through the air permeable waterproof sheet 19.

The injection order of the glucose solution B and the alkaline solution A may be reverse, and may be simultaneous.

At this time point, because the manual valve 71 is in a closed state, the oxidation electrode 85 and the reduction electrode 86 are electrochemically isolated from each other, and an oxidation-reduction reaction does not occur. The glucose has such properties as to be isomerized in an alkaline environment, but at this time point, both of the solutions are separated from each other, and accordingly the isomerization of the glucose can be prevented.

Next, as illustrated in Fig. 37, two manual valves 71 are simultaneously opened by the link 77, thereby the glucose solution B and the alkaline solution A are mixed to each other in a first cell constituted by the first oxidation electrode chamber 81 and the first reduction electrode chamber 83 and a second cell constituted by the second oxidation electrode chamber 82 and the second reduction electrode chamber 84, and the oxidation-reduction reaction occurs in each cell. Thereby, the battery is structured as a whole so that the first cell and the second cell are electrically serially connected.

When a battery output has declined, the drain cock 76 is opened as preparation for the operation of replacing the fuel liquid, and the mixed liquid of the glucose solution B and the alkaline solution A is discharged, as illustrated in Fig. 39.

At this time, as illustrated in Fig. 38, when the syringe needle 27 is inserted into any one of the injection port 78 and the injection port 79, and air is injected into the first oxidation electrode chamber 81 or the first reduction electrode chamber 83, the solution which has been extruded by the pressure is discharged through each of the non-return valves 73 and the drain cock 76.

In the present embodiment, the structure can also be employed in which the fuel bag or the periphery of the electrode is integrated, as in the first or the second embodiment, for instance.

[Ninth embodiment]

Next, a battery 7 according to a ninth embodiment of the present invention will be described with reference to the drawings. In the description of the present embodiment, the description concerning common points to each of the above described embodiments will be omitted, and a different point will be mainly described below.

The battery 7 according to the present embodiment is, for instance, a direct methanol type fuel cell.

As illustrated in Fig. 40, an inner part of a container 10 is divided by partition walls 97 and 98 to form a first chamber 91, a second chamber 92 and a third chamber 93 formed therein.

In the structure according the present embodiment, the structure may also be employed in which the fuel bag or the periphery of the electrode is integrated, as in the first or the second embodiment, for instance.

Apertures are provided in bottom faces of the first chamber 91, the second chamber 92 and the third chamber 93, respectively, and solid electrolyte membranes 96 are provided on these apertures, respectively.

On the inside of each solid electrolyte membrane 96, a carbon fiber electrode having platinum fine particles fixed thereon is arranged as a negative electrode 95, respectively.

In addition, on the outside of each solid electrolyte membrane 96, a carbon fiber electrode having platinum fine particles fixed thereon is arranged as a positive electrode 94, respectively.

A positive electrode terminal 21 is connected to the positive electrode 94 of the first chamber 91, and a negative electrode terminal 22 is connected to the negative electrode 95 of the third chamber 93. The negative electrode 95 of the first chamber 91 and the positive electrode 94 of the second chamber 92 are connected by a conducting wire 23. The negative electrode 95 of the second chamber 92 and the positive electrode 94 of the third chamber 93 are connected by a conducting wire 23. By being structured in this way, the first chamber 91, the second chamber 92 and the third chamber 93 are structured so as to be serially connected, and supply the electric power to electronic equipment which has been connected to the positive electrode terminal 21 and the negative electrode terminal 22.

Apertures are provided on upper faces of the first chamber 91, the second chamber 92 and the third chamber 93, respectively.

An air permeable waterproof sheet 19 is provided on each of the apertures of the first chamber 91 and the third chamber 93. On the other hand, an injection port 99 for injecting a fuel liquid (methanol) therethrough is provided in the aperture of the second chamber 92.

A non-return valve 100 is provided in the partition wall 97, which permits the fluid to flow in the direction from the second chamber 92 to the first chamber 91, and on the other hand, prohibits the fluid to flow in the direction from the first chamber 91 to the second chamber 92.

The non-return valve 100 is provided in the partition wall 98, which permits the fluid to flow in the direction from the second chamber 92 to the third chamber 93, and on the other hand, prohibits the fluid to flow in the direction from the third chamber 93 to the second chamber 92.

A function of the battery 7 having the above described structure will be described below.

Firstly, as illustrated in Fig. 41, a syringe needle 27 is inserted into the injection port 99 in a state where a fuel liquid is contained in the syringe 9, and the fuel liquid is injected into the second chamber 92 from the syringe 9.

Thereby, the pressure in the second chamber 92 rises, each of the non-return valves 100 is opened by this pressure, and the fuel liquid flows into each of the first chamber 91 and the third chamber 93 from the second chamber 92.

At this time, a gas which has originally filled the inner part of each chamber is extruded by the pressure of the fuel liquid to be injected, and is exhausted from the air permeable waterproof sheet 19 provided in each of the first chamber 91 and the third chamber 93.

After the injection operation to all of the chambers has been finished, the syringe needle 27 is extracted. Thereby, the injection port 99 and each non-return valve 100 are closed, and the first chamber 91, the second chamber 92 and the third chamber 93 are isolated from each other as each independent cell. Thereby, the battery is structured as a whole so that the first chamber 91, the second chamber 92 and the third chamber 93 are electrically serially connected.

[Tenth embodiment]

Next, a battery 8 according to a tenth embodiment of the present invention will be described with reference to the drawings. In the description of the present embodiment, the description concerning common points to the above described embodiment will be omitted, and a different point will be mainly described below.

The point at which the battery 8 according to the present embodiment is different from the battery according to each of the above described embodiments is a structure of electrically connected cells. Incidentally, the concept of each of the above described embodiments can be applied to the container, the partition wall, the positive electrode, the negative electrode, the injection port, a continuous hole and a flow-channel opening/closing portion, which are structures except the structure of the electrically connected cells.

Fig. 42 is a schematic view describing the structure of the electrically connected cells of the battery 8 according to the present embodiment.

As illustrated in Fig. 42, the battery 8 according to the present embodiment has a plurality of cells (cell A to cell E) which contain an electrolyte fluid therein. The positive electrode 14 and the negative electrode 15 are arranged in the inner part of each of the cell A to the cell E.

Each of the cell A to the cell E communicates with a common flow channel 102 through a flow-channel opening/ closing portion 103, for instance, such as a manual valve.

An injection port 101 for injecting the electrolyte fluid therethrough is provided in the common flow channel 102.

Each of the positive electrodes 14 and the negative electrodes 15 which are arranged in the inner part of the cell A to the cell E is connected to an electrical-connection switching device (electrical-connection switching portion) 104. The electrical-connection switching device 104 is a device which switches the connection structure of the positive electrode 14 and the negative electrode 15 in each cell. Devices A to C are connected to the cell A to the cell E through this electrical-connection switching device 104.

In the example illustrated in Fig. 42, the positive electrode 14 of the cell A and the negative electrode 15 of the cell B are connected to the device A, and the negative electrode 15 of the cell A and the positive electrode 14 of the cell B are also connected. Specifically, a power source of the device A has a structure in which the cell A and the cell B are serially connected.

The positive electrode 14 and the negative electrode 15 of the cell C are connected to the device B. Specifically, the cell C is independently provided as the power source of the device B.

The positive electrode 14 of the cell D and the negative electrode 15 of the cell E are connected to the device C, the positive electrode 14 of the cell D and the positive electrode 14 of the cell E are also connected, and the negative electrode 15 of the cell D and the negative electrode 15 of the cell E are connected. Specifically, the power source of the device C has a structure in which the cell D and the cell E are connected in parallel.

By having the above described structure, the battery 8 according to the present embodiment can flexibly select and use a combination of cells in parallel connection or serial connection as an individual battery for each of a plurality of the devices, though being a single battery in which the fuel liquid has been injected into the plurality of the cells.

When the power source of the device C has the structure in which the cell D and the cell E are connected in parallel, the flow-channel opening/closing portions 103 of the cell D and the cell E may be opened. In this case, the fluid can be convected between the cell D and the cell E, and accordingly such an effect that the state of the fluid is averaged is additionally produced.

In the present embodiment, the electrical connection structure for the devices A to C can be easily changed by operating the electrical-connection switching device 104. For instance, it is also possible to connect the cell A and the cell B to the device A in parallel, and to connect the cell D and the cell E to the device C in series. It is also possible to connect all of the cells to each device in parallel or in series.

In the structure according the present embodiment, the structure may also be employed in which the fuel bag or the periphery of the electrode is integrated, as in the first or the second embodiment, for instance.

[Eleventh embodiment]

Next, a battery 9 according to an eleventh embodiment of the present invention will be described with reference to the drawings. In the description of the present embodiment, the description concerning common points to each of the above described embodiments will be omitted, and a different point will be mainly described below.

The point at which the battery 9 according to the present embodiment is different from the battery 8 according to the above described tenth embodiment is a method of electrically connecting cells.

As illustrated in Fig. 43, the electrical-connection switching device 104 has switches A to E which are connected to the positive electrodes 14 of the cell A to the cell E, respectively.

In the battery 9 according to the present embodiment, the positive electrodes 14 of the cell A to the cell E are connected through the switches A to E respectively, and are connected to a device 105. The negative electrodes 15 of the cell A to the cell E are connected respectively, and are connected to the device 105. Specifically, the power source of the device 105 has a structure in which the cells A to E are connected in parallel through the switches A to E.

In the example illustrated in Fig. 43, the switch A is closed, but the other switches are opened. In this case, the electric power is supplied to the device 105 only from the cell A.

When the electric power of the cell A has been depleted by the operation of the device 105, the structure can be switched to a structure illustrated in Fig. 44. In Fig. 44, the switch B is closed, but the other switches are opened. By this switching operation, the use of the cell A in which the electric power has been depleted is stopped, and the use of the unused cell B can be started.

The battery 9 according to the present embodiment is effective in the structure in which each cell is serially connected similarly to that in each of the above described embodiments, but also in the case when each cell is applied to parallel connection, the same effect as in the case when the battery is replaced with a new one can be obtained, by switching the structure to a structure in which the device is connected to the unused cell, when the electric power of the used cell has been depleted as described above.

Each of the embodiments according to the present invention has been described in detail above with reference to the drawings, but the specific structure is not limited to this embodiment, and the change in a design is also included in such a range as not to deviate from the scope of the invention.

For instance, in each embodiment, the number of the cells formed in the container 10 is not limited to these examples, and the present invention can be applied to each embodiment as long as the structure has two or more cells formed therein.

In each embodiment, examples have been described while taking an enzyme type glucose-fuel cell, an alkali type glucose-fuel cell and the like, but the type of the battery is not limited to these examples, and the present invention can be applied to any battery as long as the battery is a type in which a fluid is injected into the battery from the outside.

In the structure according to the present embodiment, the structure may also be employed in which the fuel bag or the periphery of the electrode is integrated, as in the first or the second embodiment, for instance.

[Thirteenth embodiment]

An electrode 301 for a fuel cell and the fuel cell 300 according to a thirteenth embodiment of the present invention will be described below with reference to Fig. 45 to Fig. 48.

The electrode 301 for the fuel cell according to the present embodiment is an electrode to be provided in the fuel cell which uses a sugar solution as a fuel; and includes a proton-conducting membrane 302, and a positive electrode 303 and a negative electrode 304 which are arranged so as to face to each other while sandwiching the proton-conducting membrane 302 therebetween, where the membrane and the electrodes are integrally formed, as illustrated in Fig. 45.

There are two types of ion-conducting membranes 302, depending on the techniques of structuring the battery. One is the case in which a cation permeable membrane 302a that makes the cation permeate therein is used, and the other is the case in which an anion permeable membrane 302b that makes the anion permeate therein is used. The above difference is a difference concerning the selection of a material, and the structure is the same. Accordingly, a case will be described below in which the cation permeable membrane 302a is used as the ion-conducting membrane 302.

The proton-conducting membrane 302 is a high-polymer membrane superior in ion conductivity, and membranes of cellophane, perfluorosulfonic acid and the like are used. The membrane of perfluorosulfonic acid preferably includes, for instance, Nafion (registered trademark) made by DuPont. The proton-conducting membrane 302a conducts the proton between a space in a positive electrode 303 side and a space in a negative electrode 304 side while dividing the space in the positive electrode 303 side and the space in the negative electrode 304 side.

The anion permeable membrane 302b is a high-polymer membrane, which is superior in conductivity for anions, and which includes such as an ammonium group, a pyridinium group, an imidazolium group, a phosphonium group and a sulfonium group; and includes NEOSEPTA (registered trademark) made by Tokuyama Corporation. The anion permeable membrane 302b conducts a hydroxy ion while dividing two electrodes similarly to the above description.

The positive electrode 303 is a sheet-shaped porous body formed of carbon or titanium, and carries fine particles of a noble metal thereon which catalyzes a reduction reaction of oxygen. The positive electrode 303 has one flat surface which is arranged so as to come in close contact with the proton-conducting membrane 302.

The negative electrode 304 is a porous body formed of carbon or titanium, and carries fine particles of a noble metal thereon which catalyzes an oxidation reaction of sugar, in such a state that the fine particles are dispersed over the whole surface.

In addition, the negative electrode 304 has one face in which a hollow is formed in such a state that an edge portion around the whole perimeter projects higher than other portions. The hollow side is arranged so as to face the proton-conducting membrane 302 side, and the edge portion comes in close contact with the proton-conducting membrane 302. Thereby, a gap A is formed between the negative electrode 304 and the proton-conducting membrane 302, and the sugar solution which has been supplied to the negative electrode 304 fills the gap A as well. The gap A is preferably formed at all positions which are adjacent to the positive electrode 303 in a state of sandwiching the proton-conducting membrane 302 therebetween. Thereby, the whole face of the positive electrode 303 on the negative electrode 304 side comes in contact with the sugar solution through the proton-conducting membrane 302, and the proton emitted by the oxidation reaction of the sugar on the negative electrode 304 is efficiently transmitted to the positive electrode 303 through the sugar solution and the proton-conducting membrane 302.

The negative electrode 304, the positive electrode 303 and the proton-conducting membrane 302 are integrally formed, for instance, by applying lamination processing to side faces of these electrodes and the membrane.

The structure and the function of the fuel cell 300 provided with the electrode 301 which is structured for the fuel cell in this way will be described below.

The fuel cell 300 according to the present embodiment includes a first current collector 305 which covers the positive electrode 303, a second current collector 306 which covers the negative electrode 304, and a fuel tank 307 provided on the negative electrode 304 side, as illustrated in Fig. 46.

The first current collector 305 and the second current collector 306 are connected to an external circuit which is not illustrated, through terminals 305a and 306a, respectively. Thereby, an electron is transmitted between the negative electrode 304 and the positive electrode 303, via the external circuit. An air supply hole 305b or a fuel supply hole 306b is penetratingly formed in the first current collector 305 and the second current collector 306, respectively. On the positive electrode 303 side, the air in the outside is supplied to the positive electrode 303 through an inner part of the air supply hole 305b. On the negative electrode 304 side, the sugar solution supplied from a fuel introduction port 308 into the fuel tank 307 is supplied to the negative electrode 304 through an inner part of the fuel supply hole 306b, is further supplied to the gap A between the negative electrode 304 and the proton-conducting membrane 302 while passing through an inner part of the negative electrode 304, and is sequentially discharged from a discharge port 309. At this time, the sugar solution is supplied from the fuel introduction port 308 at a sufficiently low speed so that turbulence does not occur in the flow of the sugar solution.

According to the fuel cell 300 structured in this way, the oxidation reaction of the sugar is conducted in the negative electrode 304, and a proton and an electron are emitted from the sugar. The emitted electron is transmitted from the negative electrode 304 to the external circuit through the second current collector 306 and the terminal 306a, and is subsequently transmitted to the positive electrode 303 through the terminal 305a and the first current collector 305. On the other hand, the emitted proton is transmitted from the negative electrode 304 to the proton-conducting membrane 302, by water molecules in the sugar solution which fills the gap A. The positive electrode 303 receives the electron from the first current collector 305, receives the proton from the proton-conducting membrane 302, and reduces oxygen in the air to produce water. Thereby, the electron which moves between the terminals 305a and 306a, specifically an electric current, can be used for an action of the external circuit.

In this case, in the conventional fuel cell, the negative electrode has been brought into close contact with the proton-conducting membrane in order to efficiently transmit the proton generated in the negative electrode to the proton-conducting membrane. In the case of such a structure, more fuel stays in the negative electrode as the position is closer to the proton-conducting membrane, and a new fuel is not easily supplied from the outside, and accordingly it has not been possible to effectively utilize a part of the negative electrode for electric power generation. Particularly, in the case of the sugar solution, the sugar has a high molecular weight, is a nonelectrolyte, and accordingly has high viscosity. Therefore, it is difficult for the sugar solution to cause self diffusion and self convection compared to other fuels such as ethanol and a gas. Accordingly, such a phenomenon has been remarkable that the oxidation reaction of the sugar in the negative electrode retards with the progress of time to lower a power generation efficiency, and the output current decreases.

However, according to the present embodiment, the face on the side of the proton-conducting membrane 302 of the negative electrode 304 which has been conventionally blocked by the proton-conducting membrane 302 is also opened to the fuel, and the sugar diffuses and migrates between the inner part and the outside of the negative electrode 304 even at the face. Thereby, the sugar already oxidized in the inner part of the negative electrode 304 can be smoothly replaced by the unreacted sugar in the outside of the negative electrode 304, and the oxidation reaction of the sugar is constantly and actively conducted at each position on the negative electrode 304 including the region which has not conventionally contributed to the electric power generation effectively. Consequently, the fuel cell according to the present embodiment shows an advantage of being capable of providing a stable and high output current while keeping the power generation efficiency at a high state.

In addition, the conventional fuel cell which has used a gas or an alcohol as the fuel has made the fuel forcibly flow, in order to efficiently replace the reacted fuel with the new fuel. On the other hand, it is necessary to form an electric double layer on the surface of each electrode 303 and 304 and maintain a potential difference between the positive electrode 303 and the negative electrode 304, in order to pass an electric current between the terminals 305a and 306a. When the sugar solution is used as the fuel, the capacitance of the electric double layer on the negative electrode 304 depends on the concentration gradient of the sugar.

Then, if the sugar solution is forcibly made to flow in the fuel cell 300, the electric double layer collapses because it takes time for the sugar having a large diffusion coefficient to form the concentration gradient, and the potential difference between the electrodes 303 and 304, specifically, an electromotive force of the fuel cell 300 decreases. As a result, the electric current which can be taken out to an external circuit decreases.

However, the fuel cell according to the present embodiment replaces the fuel by the self diffusion of the fuel without depending on the forced flow of the fuel, and thereby stably holds the electric double layer of the negative electrode 304 even if the sugar solution is used as the fuel. Thereby, the fuel cell 300 has an advantage of being capable of stably generating the electric power while maintaining the electromotive force in a high state.

In the above described embodiment, the gap A has been formed between the negative electrode and the proton-conducting membrane 302 by using the negative electrode 304 having a cross section with a lateral U shape, but in place of the above configuration, the gap A may also be formed between the negative electrode 304 and the proton-conducting membrane 302 by providing a spacer 310 between the sheet-shaped negative electrode 304 and the proton-conducting membrane 302, as illustrated in Fig. 47. When the spacer 310 is formed from a material which does not make the sugar solution permeate therethrough, a continuous hole 310a which makes the gap A communicate with the discharge port 309 is formed in the spacer 310. By being structured in this way, the electrode 301 for the fuel cell can be manufactured by using the sheet-shaped electrode which has been conventionally used as the negative electrode, in an intact state as the negative electrode 304.

In the above described embodiment, a fuel supply hole 306b has been formed in the second current collector 306 so as to supply the fuel in the fuel tank 307 to the negative electrode 304. However, in place of the above configuration, the face of the fuel tank 307 side of the negative electrode 304 may be covered with a porous member 311 which makes the sugar solution permeate therethrough, as illustrated in Fig. 48. The porous member 311 is formed from a material which does not affect the oxidation reaction of the sugar in the negative electrode 304 while having superior electroconductivity. Thereby, the sugar solution can be uniformly supplied to each position of the negative electrode 304.

The ion-conducting membrane according the present embodiment may be used for the cell structure including the electrode portion and the electrode periphery in the first embodiment.

[Fourteenth embodiment]

Next, the electrode 301 for the fuel cell and the fuel cell 300 according to a fourteenth embodiment of the present invention will be described with reference to Fig. 49 to Fig. 53. In the present embodiment, a different point from the thirteenth embodiment will be mainly described. Concerning a structure in common with the thirteenth embodiment, the same reference numerals will be put on the structure, and the descriptions will be omitted.

The electrode 301 for the fuel cell according to the present embodiment differs from the thirteenth embodiment in the structure of the negative electrode 304.

The negative electrode 304 is formed to have a fin shape in which one surface extends approximately perpendicularly with respect to the other flat surface, as illustrated in Fig. 49. The height of the fin 312 is appropriately adjusted so that the sugar can easily move between the inner part and the outside of a groove formed between the fins 312, by natural convection.

The electrode 1 for the fuel cell is provided in the fuel cell 300 so that the fin 312 faces to the fuel tank 307 side, and the position of the groove formed between the fins 312 matches the position of the fuel supply hole 306b, as illustrated in Fig.50.

According to the fuel cell 300 structured in this way, the fin 312 is formed on the surface of the negative electrode 304 to increase the exposed surface area of the negative electrode 304, and thereby the migration of the sugar between the inner part and the outside of the negative electrode 304 is promoted by the diffusion migration of the sugar. Thereby, the already oxidized sugar is smoothly replaced with the unreacted sugar, the power generation efficiency is maintained, and the highly stable output current can be obtained. In addition, the flat surface of the negative electrode 304 is arranged so as to come in close contact with the proton-conducting membrane 302, and accordingly even if not only the sugar solution but also a fuel of non-ion conductivity such as a gas and ethanol is used, the proton is efficiently transmitted from the negative electrode 304 to the proton-conducting membrane 302. Accordingly, even if the fuel other than the sugar solution is used, the power generation efficiency is similarly enhanced, and the stable output current can be obtained.

Incidentally, in the above described embodiment, the negative electrode 304 has been arranged so that the flat face faces the proton-conducting membrane 302 side and comes in close contact with the proton-conducting membrane 302, but in place of the above configuration, the fin 312 may be arranged so as to face to the proton-conducting membrane 302 side, or may be arranged so as to form a space between the negative electrode and the proton-conducting membrane 302. By being structured in this way, the face on the proton-conducting membrane 302 side of the negative electrode 304 is also opened to the fuel similarly to the thirteenth embodiment to further promote the diffusion migration of the sugar between the inner part and the outside of the negative electrode 304, and thereby the whole negative electrode 304 can be effectively utilized for the electric power generation to further enhance the power generation efficiency.

In the above described embodiment, the negative electrode 304 having the fin 312 on one face has been taken as the example, but the shape of the negative electrode 304 is not limited to this, and any shape is acceptable as long as the exposed surface area exposed to the fuel increases. Other examples of the negative electrode 304 are illustrated in Fig. 51 to Fig. 53.

The negative electrode 304 illustrated in Fig. 51 has the fin 312 formed on both faces. In this case, the negative electrode 304 has shallow grooves between each fin 312 while maintaining the same volume and the exposed surface area of the negative electrode 304, in comparison with the case in which the fin 312 is formed on one face, accordingly further promotes the natural convection of the fuel, and can further enhance the electric power generation capacity.

The negative electrode 304 illustrated in Fig. 52 has slits 313 formed thereon in a lattice shape. In this case as well, the slit 313 may be formed on both faces similarly to the negative electrode 304 having the fin 312.

The negative electrode 304 illustrated in Fig. 53 is formed so as to have unit blocks 314 arranged checkwise in a three-dimensional direction. In this case, it is preferable to approximately equalize the dimension of the unit blocks 314 with the dimension of the gaps between the unit blocks 314 so that the fuel easily migrates by convection also in the gaps between the unit blocks 314.

By being structured in this way as well, the negative electrode 304 increases the exposed surface area to promote the replacement of the already oxidized fuel with the unreacted fuel, thereby utilizes the whole negative electrode 304 effectively for the electric power generation, and can provide a stable and high output current even if the sugar solution is used as the fuel. In addition, when the negative electrode 304 is arranged so as to form a space between the negative electrode and the proton-conducting membrane 302, the arrangement can further enhance the power generation efficiency.

Furthermore, the electrode according to the present embodiment may also be used for the electrode of the fuel cell, for instance, according to the first embodiment.

{Reference Signs List}

1, 2, 3, 4, 5, 6, 7, 8, 9    Battery

9                            Syringe

10                           Container

| 11, 12 | Cell |
| 13 | Partition wall |
| 14 | Positive electrode |
| 15 | Negative Electrode |
| 16 | Injection port |
| 17 | Slit valve (Flow-channel opening/closing portion) |
| 18 | Continuous hole |
| 19 | Air permeable waterproof sheet |
| 25 | Slit |
| 27 | Syringe needle |
| 29 | Pinhole |
| 51 | Injection port |
| 52 | Manual cock |
| 53 | Manual valve |
| 60 | Common flow channel |
| 61 | Cell |
| 67 | Non-return valve |
| 71 | Manual valve |
| 77 | Link (Connection mechanism) |
| 101 | Injection port |
| 102 | Common flow channel |
| 103 | Flow-channel opening/closing portion |
| 104 | Electrical-connection switching device |
| 201, 202, 203 | Fuel cell |
| 211, 251 | Container |
| 212, 252 | Fuel bag (Storage portion) |
| 213, 253 | Injection port |
| 214, 254 | Discharge port |
| 217 | Aeration portion |

| 218, 219, 258 | Septum (Injection/discharge port) |
| 220 | Electrode |
| 221, 222 | Cathode |
| 225, 226 | Anode |
| 250 | Partition wall |
| 261, 262 | Fin (Heat exchanger) |
| 271, 272 | Piping (Flow channel) |
| 301 | Electrode for fuel cell |
| 302 | Proton-conducting membrane |
| 303 | Positive electrode |
| 304 | Negative Electrode |
| 305 | First current collector |
| 305a, 306a | Terminal |
| 305b | Air supply hole |
| 306 | Second current collector |
| 306b | Fuel supply hole |
| 307 | Fuel Tank |
| 308 | Fuel introduction port |
| 309 | Discharge port |
| 310 | Spacer |
| 310a | Continuous hole |
| 311 | Porous member |
| 312 | Fin |
| 313 | Slit (Groove) |
| 314 | Unit block |
| 300 | Fuel cell |

**Claims**

1. A fuel cell comprising:

   a container which contains an electrolyte solution therein;

a pair of electrodes arranged in the container;

an aeration portion which is formed on at least one part of an outer surface of the container and has air permeability and waterproofness; and

an injection/discharge port for injecting a fuel from the outside into the container or discharging the fuel from the container.

2. The fuel cell according to claim 1, further comprising:

a storage portion for storing a fuel supplied from the outside therein; and

a flow channel for connecting the container to the storage portion.

3. The fuel cell according to claim 2, wherein the injection/discharge port is provided on an outer surface of at least one of the container and the storage portion.

4. The fuel cell according to claim 2 or 3, wherein

a partition wall is provided in an inner part of the storage portion so as to divide the storage portion into one face side in which the injection/discharge port is provided and another face side which opposes the one face and so as to be opened in an end edge, and

the flow channel is connected to each division.

5. The fuel cell according to claim 4, further comprising a heat exchanger which exchanges heat between the outside and the inside of the storage portion, provided on an outer surface of the storage portion.

6. The fuel cell according to any one of claims 1 to 5, wherein the aeration portion is formed of a carbon fluoride resin.

7. The fuel cell according to claim 6, wherein

a wall of the container is formed of a carbon fluoride resin, and

the aeration portion is a portion in which the wall of the container is formed so as to be locally thin.

8. A battery comprising:

a container which contains an electrolyte fluid therein;

a partition wall for dividing the container and forming a plurality of cells in the container;

a positive electrode and a negative electrode arranged in each of the cells, respectively;

an injection port provided in the container, through which the electrolyte fluid is injected into the container from the outside;

a continuous hole which is provided in the partition wall and makes each of the cells communicate with each other; and

a flow-channel opening/closing portion which is provided in the continuous hole and opens/closes a flow channel between each of the cells.

9. The battery according to claim 8, wherein the flow-channel opening/closing portion opens the flow channels between each of the cells when the electrolyte fluid is injected into the container, and closes the flow channels between each of the cells after the electrolyte fluid has been injected into the container.

10. The battery according to claim 8, wherein the flow-channel opening/closing portion is arranged on one straight line which passes the injection port and the continuous hole, and is an elastic body having a slit therein.

11. The battery according to claim 8, wherein the flow-channel opening/closing portion is a valve which opens/closes the flow channels between each of the cells.

12. The battery according to claim 11, further comprising a plurality of the valves, and

a connection mechanism which connects the plurality of the valves with each other.

13. The battery according to claim 8, wherein the flow-channel opening/closing portion is a non-return valve that passes the electrolyte fluid in one direction from the cell to which the electrolyte fluid is injected, to other cells.

14. The battery according to claim 8, wherein

the partition wall forms a common flow channel which is adjacent to each of the cells,
the injection port is provided in the common flow channel, and
the continuous hole and the flow-channel opening/closing portion are provided in the partition wall which separates the common flow channel from each of the cells.

15. The battery according to claim 14, wherein each of the cells is arranged so as to be adjacent in the outside of the common flow channel.

16. The battery according to claim 8, further comprising a flow channel formed therein which makes the single injection port communicate with the plurality of the cells,
on the condition that the continuous hole is opened by the flow-channel opening/closing portion.

17. The battery according to claim 8, further comprising an electrical-connection switching portion provided therein which switches an electrical connection between the positive electrode and the negative electrode in each of the cells.

18. An electrode for a fuel cell, comprising:

a porous negative electrode which oxidizes a fuel;
a positive electrode which reduces oxygen; and
an ion-conducting membrane which interposes between the negative electrode and the positive electrode,

wherein the negative electrode is arranged so as to have a gap between the negative electrode and the ion-conducting membrane.

19. An electrode for a fuel cell comprising:

a porous negative electrode which oxidizes a fuel;
a positive electrode which reduces oxygen; and
an ion-conducting membrane which interposes between the negative electrode and the positive electrode,
wherein the negative electrode has asperity formed on a surface thereof.

20. The electrode for the fuel cell according to claim 19, wherein the negative electrode has the surface formed into a fin shape.

21. The electrode for the fuel cell according to claim 19, wherein the negative electrode has a groove formed on the surface.

22. The electrode for the fuel cell according to claim 19, wherein the negative electrode is arranged so as to have a gap between the negative electrode and the ion-conducting membrane.

23. A fuel cell provided with the electrode for the fuel cell according to any one of claims 18 to 22.

24. The fuel cell according to claim 23, wherein the ion-conducting membrane is a cation permeable membrane.

25. The fuel cell according to claim 23, wherein the ion-conducting membrane is an anion permeable membrane.

FIG. 1

FIG. 2

# FIG. 3

## FIG. 4

232

220

231

## FIG. 5

232

223

221

227

223

222

228

225    226

## FIG. 6

$2e^-$

$2e^-$

$C_6H_{12}O_6$

$2e^-$

$2H^+$

$^{1/2}O_2$

$C_6H_{10}O_6$

$H_2O$

負極

正極

## FIG. 7

202

254

250

251

261

252

258

259 253

FIG. 8

FIG. 9

## FIG. 10

## FIG. 11

# FIG. 12

# FIG. 13

# FIG. 14

# FIG. 15

# FIG. 16

FIG. 17

FIG. 18

# FIG. 19

# FIG. 20

FIG. 21

FIG. 22

# FIG. 23

# FIG. 24

FIG. 25

EP 2 490 288 A1

# FIG. 26

EP 2 490 288 A1

FIG. 27

# FIG. 28

# FIG. 29

FIG. 30

FIG. 31

FIG. 32

FIG. 33

# FIG. 34

# FIG. 35

# FIG. 36

# FIG. 37

# FIG. 38

# FIG. 39

# FIG. 40

# FIG. 41

# FIG. 42

EP 2 490 288 A1

FIG. 43

# FIG. 44

EP 2 490 288 A1

# FIG. 45

301

304

302

303

# FIG. 46

307

306b 306b    306b   306b 306b

308

309

306

(−)

A

304

306a

302

303

305

(+)

305a

305b    305b    305b    305b

# FIG. 47

# FIG. 48

## FIG. 49

304    312

## FIG. 50

400

307    312    312    312    308
306b    306b    306b    306b    306b
309
306    306a
301    304
302
303    305a
305
305b    305b    305b    305b

## FIG. 51

## FIG. 52

304

# FIG. 53

314

## INTERNATIONAL SEARCH REPORT

| International application No. |
| --- |
| PCT/JP2010/066403 |

A. CLASSIFICATION OF SUBJECT MATTER
*H01M8/04*(2006.01)i, *H01M8/02*(2006.01)i, *H01M8/08*(2006.01)i

According to International Patent Classification (IPC) or to both national classification and IPC

B. FIELDS SEARCHED

Minimum documentation searched (classification system followed by classification symbols)
H01M8/04, H01M8/02, H01M8/08

Documentation searched other than minimum documentation to the extent that such documents are included in the fields searched
Jitsuyo Shinan Koho          1922-1996   Jitsuyo Shinan Toroku Koho   1996-2010
Kokai Jitsuyo Shinan Koho    1971-2010   Toroku Jitsuyo Shinan Koho   1994-2010

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 42-3658 B1  (AG. Buraun Poberi & Co.), 16 February 1967 (16.02.1967), (Family: none) | 1-7 |
| A | JP 41-16011 B1  (Esso Research and Engineering Co.), 09 September 1966 (09.09.1966), (Family: none) | 1-7 |
| A | Microfilm of the specification and drawings annexed to the request of Japanese Utility Model Application No. 138015/1987(Laid-open No. 43553/1989) (Hitachi Maxell, Ltd.), 15 March 1989 (15.03.1989), (Family: none) | 1-7 |

☒  Further documents are listed in the continuation of Box C.          ☐   See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search 24 December, 2010 (24.12.10) | Date of mailing of the international search report 11 January, 2011 (11.01.11) |
| --- | --- |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

| International application No. |
|---|
| PCT/JP2010/066403 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 63-76269 A (Hitachi Maxell, Ltd.), 06 April 1988 (06.04.1988), (Family: none) | 1-7 |
| A | JP 2-148657 A (Matsushita Electric Industrial Co., Ltd.), 07 June 1990 (07.06.1990), (Family: none) | 1-7 |
| A | JP 2008-282586 A (Sony Corp.), 20 November 2008 (20.11.2008), paragraph [0047] & US 2008/0280184 A1 | 1-7 |
| A | WO 2009/072564 A1 (Sony Corp.), 11 June 2009 (11.06.2009), paragraph [0110] & JP 2009-158466 A    & WO 2010/007833 A1 | 1-7 |
| A | JP 2009-140646 A (Sony Corp.), 25 June 2009 (25.06.2009), (Family: none) | 1-7 |

Form PCT/ISA/210 (continuation of second sheet) (July 2009)

**INTERNATIONAL SEARCH REPORT**

International application No.

PCT/JP2010/066403

| Box No. II | Observations where certain claims were found unsearchable (Continuation of item 2 of first sheet) |

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |

This International Searching Authority found multiple inventions in this international application, as follows:
   As described in (extra sheet), In order that a group of inventions set forth in claims comply with the requirement of unity, it is required that a special technical feature for so linking the group of inventions as to form a single general inventive concept is present. However, it is considered that three inventions, which are classified into claims 1 - 7, claims 8 - 17, and claims 18 - 25, are set forth in claims of the present international application.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1 - 7

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2010/066403

Continuation of Box No.III of continuation of first sheet(2)

In order that a group of inventions set forth in claims comply with the requirement of unity, it is required that a special technical feature for so linking the group of inventions as to form a single general inventive concept is present, but, the inventions set forth in claims 1 - 25 are considered to be linked one another by only the matter such as "electrode for a fuel cell".

However, it is obvious that the above-said matter cannot be a special technical feature without necessity for presentation of the prior art documents.

Consequently, among a group of inventions set forth in claims 1 - 25, there is no special technical feature for so linking the inventions as to form a single general inventive concept. Therefore, it is obvious that the group of inventions set forth in claims 1 - 25 does not comply with the requirement of unity of invention.

Furthermore, a group of inventions set forth in claims 1 - 17 is considered to be linked to one another by only the matter such as "cell comprising a container for storing an electrolytic fluid, and a pouring port provided to said container".

However, the above-said matter cannot be a special technical feature, since said matter is described in, for example, JP 2008-282586 A (Sony Corp.), 20 November 2008 (20.11.2008), paragraph 0047, WO 2009/072564 A1 (Sony Corp.), 11 June 2009 (11.06.2009), paragraph 0110, etc.

From the specific modes of the inventions set forth in independent claims, it is considered that three inventions, which are classified into claims 1 - 7, claims 8 - 17 and claims 18 - 25, are set forth in claims of the present international application.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008270206 A
- JP 2004012811 PCT
- JP 2008282586 A
- JP 3518461 B

**Non-patent literature cited in the description**

- *Information energy device having biological function,* July 2009
- Concentration dependence of the mechanism of glucose oxidation at gold electrodes in alkaline media. *J. Electroanal. Gem.,* 1989, vol. 262, 167-182
- Preparation of gold electrode modified with pyridyl phosphonate and glucose oxidation capability thereof. *proceedings in Meeting of West Japan Branch of the Chemical Society of Japan,* 2004, 74
- Preparation of electrode with function of glucose oxidation catalyst using gold nano-particle and development of fuel cell'' and ''Preparation of electrode having glucose oxidation capability and application thereof to glucose fuel cell. *Abstracts in Meeting of the Electrochemical Society of Japan,* 2005, 212
- Practical Bioelectrochemistry, glucose-air fuel cell. CMC Publishing Co., Ltd, March 2007